# EUROPEAN PATENT SPECIFICATION

(11) **EP 3 028 049 B1**
(45) Date of publication and mention of the grant of the patent: **20.03.2019**
(21) Application number: 14744599.3
(22) Date of filing: 30.07.2014
(51) Int. Cl.: G01N 33/68, G01N 33/92

(54) **DIAGNOSTIC TOOLS FOR ALZHEIMER'S DISEASE**
DIAGNOSEINSTRUMENTE FÜR MORBUS ALZHEIMER
INSTRUMENTS DE DIAGNOSTIC POUR LA MALADIE D'ALZHEIMER

(30) Priority: 31.07.2013 EP 13178711
(43) Date of publication of application: 08.06.2016
(73) Proprietor: Pharnext, 92130 Issy les Moulineaux (FR)
(72) Inventor: COHEN, Daniel, F-92210 Saint Cloud (FR); CHUMAKOV, Ilya, F-77000 Vaux Le Penil (FR); NABIROTCHKIN, Serguei, F-92290 Chatenay Malabry (FR); GUEDJ, Mickael, F-75011 Paris (FR); HAJJ, Rodolphe, F-78100 St Germain En Laye (FR)
(74) Representative: Cabinet Becker et Associés
(86) International application number: PCT/EP2014/066414
(87) International publication number: WO 2015/014903

(56) References cited:
- EP-A1- 2 085 082
- WO-A1-2007/064784
- WO-A1-2012/168561
- WO-A1-2013/059234
- WO-A1-2013/070839
- US-A1- 2010 124 756
- JAMES D. DOECKE: "Blood-Based Protein Biomarkers for Diagnosis of Alzheimer Disease", ARCHIVES OF NEUROLOGY (AMERICAN MEDICAL ASSOCIATION), vol. 69, no. 10, 1 October 2012 (2012-10-01), page 1318, XP055094705, Chicago IL USA ISSN: 0003-9942, DOI: 10.1001/archneurol.2012.1282
- MUTH A ET AL: "Simultaneous enantioselective analysis of chiral urinary metabolites in patients with Zellweger syndrome", JOURNAL OF CHROMATOGRAPHY B: BIOMEDICAL SCIENCES & APPLICATIONS, ELSEVIER, AMSTERDAM, NL, vol. 792, no. 2, 25 July 2003 (2003-07-25) , pages 269-277, XP004434982, ISSN: 1570-0232, DOI: 10.1016/S1570-0232(03)00285-X
- EUGENIA TRUSHINA ET AL: "Identification of Altered Metabolic Pathways in Plasma and CSF in Mild Cognitive Impairment and Alzheimer's Disease Using Metabolomics", PLOS ONE, vol. 8, no. 5, 20 May 2013 (2013-05-20), page e63644, XP055176105, DOI: 10.1371/journal.pone.0063644

## Description

### FIELD OF THE INVENTION

The present invention relates generally to the fields of biology and medicine. The present invention relates in particular to methods of detecting predisposition to or diagnosis and/or prognosis of Alzheimer's disease (AD) and related disorders. More specifically, the invention relates to the development, validation and application of new biomarkers, which can be used for detecting the presence, the risk, or for predicting the severity of AD and related disorders. The novel biomarkers can be measured in biological body fluids or easily available extracts of biopsies, which can be used to aid in the detection of the disease, prediction of drug treatment and follow up of this treatment of neurodegenerative disorders, including AD. The present invention also relates to methods for identification of the stage of the disease, assessing the responsiveness to the treatment and the efficacy of treatment in subjects having AD or a related disorder.

### BACKGROUND OF THE INVENTION

AD is at present the most common cause of dementia. It is clinically characterized by a global decline of cognitive function that progresses slowly and leaves end-stage patients bound to bed, incontinent and dependent on custodial care. Death occurs, on average, 9 years after diagnosis [1]. The incidence rate of AD increases dramatically with age. United Nation population projections estimate that the number of people older than 80 years will approach 370 million by the year 2050. Currently, it is estimated that 50% of people older than age 85 years are afflicted with AD. Therefore, more than 100 million people worldwide will suffer from dementia in 50 years. The vast number of people requiring constant care and other services will severely affect medical, monetary and human resources [2].

Currently, clinical diagnosis of AD is based on structured interviews (patient histories), and neuropsychological examinations coupled with imaging or 2 neurophysiological scans (CT, MRI, PET and/or SPECT scans and EEG) to rule out other explanations of memory loss including temporary (depression or vitamin B12 deficiency) or permanent conditions (stroke) and is based on NINCDS-ADRDA Work group criteria [3] and the American Psychiatric Association Diagnostic and Statistical Manual of Mental Disorders [4].

Unfortunately, clinical diagnostic methods are not foolproof. Evidence based review of current literature shows clinical diagnostic accuracy of 65 to 90%. Higher accuracy rates are generally associated with specialized centers (memory disorder clinics) focused on memory disorders whereas lower rates are likely associated with primary care physicians. Additionally, accuracy of the clinical diagnosis is likely lower during early stages of the disease when symptoms are difficult to differentiate from normal age-associated cognitive decline. More recently, studies suggest that a condition termed Mild Cognitive Impairment (MCI) may represent in some cases a prodromal AD and, if diagnosed early, represents the best opportunity for pharmaceutical intervention. The clinical criteria used for diagnosis of MCI are those of Petersen *et al.* [5] and include: 1) memory complaints corroborated by an informant, 2) objective memory impairment for age and education, 3) normal general cognitive function, 4) intact activities of daily living, and 5) the subject does not meet criteria for dementia. This clinical criteria of MCI can be implemented with the identification of biomarkers such as those described in Albert *et al.* [6] and which are involved in neuronal injury (such as tau) and/or in Aβ deposition (such as Aβ42 in the Cerebro-Spinal Fluid). These biomarkers may be quantified through medical imaging and in the CSF. For instance, Amyvid is a FDA approved radioactive tracer that helps diagnosing AD by detecting amyloid plaques with the positron emission tomography imaging technology. This test, however, does neither allow predicting the development of AD nor measuring the response to the treatment and should only be used as an adjunct to other diagnostic evaluations to do this (FDA Press Release, April 10, 2012).

Further complicating diagnosis and treatment of AD is the lack of a reliable biomarker that specifically identifies AD subjects and those at risk for a conversion from MCI to AD, particularly early in the prodromal stage of the disease (MCI). In view of the magnitude of the public health problem posed by AD, considerable research efforts have been undertaken to elucidate the etiology of AD as well as to identify biomarkers, characteristic proteins or metabolites objectively measured as an indicator of pathogenic processes, that can be used to diagnose and/or predict whether a person is likely to develop AD.

Most studies of biomarkers for AD have focused on measurement in the cerebrospinal fluid (CSF). Because of its intimate contact with the brain, pathogenic changes in the brain that result in alterations in proteins/peptides would likely be reflected in the CSF. Beside well known TAU, amyloid precursor protein derivatives, or neuronal thread protein, some CSF proteinaceous biomarkers described in the literature are alpha-(1)-antichymotrypsin, chromoganin A, β-2-microglobulin, transthyretin, cystatin C, transferritin or protaglandin-D-synthase; other studies measured proteinaceous biomarkers in biological fluids samples as blood (for instance US2010124756) but attempts to replicate the results of these studies failed [7]. Hence, it is not possible to derive from these studies a common set of biomarkers that could be considered a signature of the disease, certainly due in part to the heterogeneity and the complexity of the disease.

Some genetic biomarkers have been identified; they are localized within genetic loci which have been identified to be responsible for most cases of familial early-onset, autosomal-dominant AD. About sporadic AD, the most important identified genetic risk factor is the *ApoE* ε4 allele: risk of developing AD is 12 times more important in homozygous people for *ApoE* ε4 [8].

Metabolites as biomarkers for AD have also been searched. For instance, reduced levels of glutamate have been found in hippocampal cells of diseased patients using magnetic resonance spectroscopy, thus putting forward this molecule as a potential specific biomarker for AD [9]. Lipofuscin-like pigments, directly measurable from blood sample of patients, have been suggested as a possible specific marker of AD [10]. Aβ peptides blood tests have also been considered; nevertheless, until now, attempts to measure Aβ peptides in blood have produced contradictory and discouraging results mainly due to the biochemical nature of Aβ peptides. Indeed, Aβ can be found free in the plasma, bound to plasma proteins, to blood cells, either under soluble, or intracellular forms or in the form of deposits, and can also be generated from the outside of the CNS. Hence, the use of Aβ plasma levels as a biomarker needs further clinical and developmental researches [11-13].

WO2010/066000 discloses several blood or urine biomarkers identified from patients suffering from several mental diseases but not from AD. WO2011/012672 discloses some metabolites from disturbed pathways in AD. WO2012/168561 discloses notably some carboxylic acids containing 2 to 5 carbon atoms, phosphatidylcholine derivatives and unidentified serum metabolites for predicting the risk of subjects of progressing to AD.

Other fluid biomarkers in AD, blood-based protein biomarkers for diagnosis of AD and biochemical markers for early diagnosis of AD are also described [14-16]. Muth et al. (2003. J. Chromatography B, 792, 269-277) discloses the analysis of chiral urinary metabolites, such as 2-hydroxysebacic acid and 3-hydroxysebacic acid, for the diagnosis of peroxisomal diseases, e.g., Zellweger syndrome.

The availability of reliable detectable biological markers would permit rapid diagnosis of AD and related diseases, patient monitoring, and efficient clinical testing of efficacy of new medications thanks to an easy monitoring of the individual response of patients to drug treatment and disease management.

### SUMMARY OF INVENTION

The present invention provides methods for diagnosing AD and related disorders. The invention stems from the identification of metabolites which represent effective biomarkers of the disease. The methods are effective, reliable, and easy to implement. They are particularly suited for diagnosing AD or related disorders from body fluids.

The object of this invention relates to a method as defined in the claims. Disclosed herein is a method for diagnosing AD or a related disorder, the method comprising determining the differential presence, in a sample from the subject, of one or more biomarker(s) selected from azelaic acid, dodecanedioic acid, hippuric acid, sebacic acid, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), tryptophan, valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe, sulfobenzylalcohol, wherein said differential presence is indicative of the presence, risk, subtype, progression or severity of said disease.

Also disclosed herein is the method comprising the combined (simultaneous or sequential) detection of several biomarkers as listed above, preferably between 2 to 10, to provide the most effective patient analysis. In this regard, disclosed herein is the method comprising determining the differential presence, in a biological sample from the subject, of:
(i) one or more biomarker(s) selected from azelaic acid, dodecanedioic acid, hippuric acid, sebacic acid, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), tryptophan, valeric acid, and
(ii) one or more biomarker(s) selected from aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe, sulfobenzylalcohol.

For example, biomarkers are selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine) and valeric acid, even more preferably PFAM (20:1), PFAM (22:1) and PFAM (22:2).

The method may be implemented with any biological sample, typically a biological fluid, such as a sample of blood, plasma, serum, urine, or CSF. The sample may be treated prior to analysis.

Disclosed herein is a method for assessing the responsiveness of a subject to a treatment for AD or a related disorder, the method comprising determining the differential presence, in a biological fluid sample from the subject, of one or more biomarker(s) as defined above, after administration of said treatment, wherein said differential presence is indicative of a subject responsive to a treatment for AD or related disorder.

Also disclosed herein is a method for monitoring the effect of a treatment in a subject having AD or a related disorder, the method comprising determining the differential presence, in a biological fluid sample from the subject, of one or more biomarker(s) as defined above, after administration of said treatment or at different point of times during the course of the treatment, wherein a correction of such differential presence during treatment is indicative of an effective treatment. The method is particularly suited for determining the response of a subject having AD to a treatment by an acetylcholinesterase (AchE) inhibitor (for instance donepezil, rivastigmine or galantamine) or an NMDA inhibitor (as memantine), or for monitoring efficacy of said treatment.

Also disclosed herein is a method of treating a subject having or suspected to have AD or a related disorder, the method comprising (i) determining the presence, risk, subtype, progression or severity of said disease in a subject using a method as defined above and, (ii) administering to the subject in need thereof, a treatment against AD or said related disorder.

Also disclosed herein is a kit comprising a capture/label agent specific for anyone of the biomarkers as defined above, for use in diagnosing AD or a related disorder in a subject.

The invention may be used in any mammalian, typically any human subject, at any stage of the disease.

### BRIEF DESCRIPTION OF FIGURES

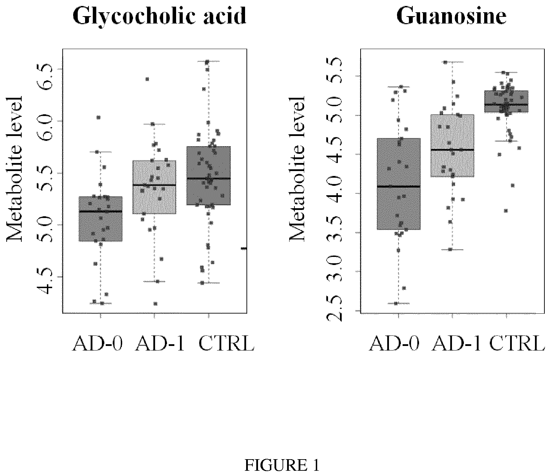

Fig. 1: Sera levels of glycocholic acid and guanosine (arbitrary logarithmic unit) in non-diseased subjects (CTRL), AD patients (AD-0) and AD patients treated with memantine (AD-1). The biomarkers level of the treated AD-1 patients is measured between the CTRL and the AD-0 levels, thereby showing a correction in the alteration of the biomarkers level.

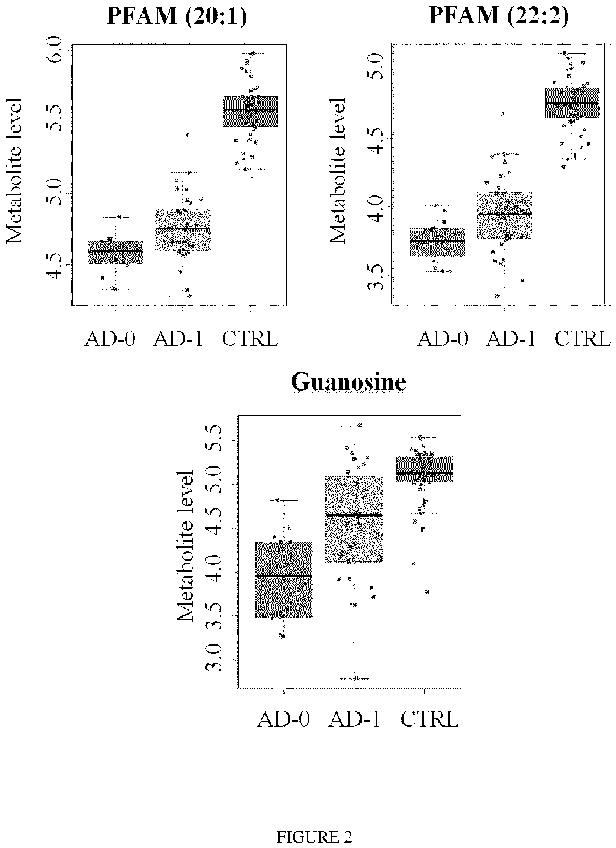

Fig. 2: Sera levels of guanosine, PFAM (20:1) and PFAM (22:2) (arbitrary unit) in non-diseased subjects (CTRL), AD patients (AD-0) and AD patients that are treated with AchE inhibitors (for instance donepezil, rivastigmine or galantamine) (AD-1). The biomarkers level of the treated AD-1 patients is measured between the CTRL and the AD-0 levels, thereby showing a correction in the alteration of the biomarkers level.

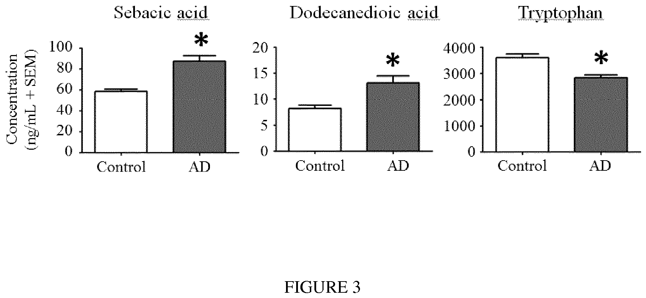

Fig. 3: Concentrations level of biomarkers in sera in AD patients and non-diseased subjects (mean +SD). Sebacic acid level is significantly increased in AD patients with a mean of 87.6 ng/mL, compared to 58.4 ng/mL. Dodecanedioic acid level is also significantly increased compared to control (means of 13.1 versus 8.2 ng/mL, respectively). Tryptophan level is significantly decreased in AD patients, with a mean concentration of 2832 ng/mL, compared to control levels of 3606 ng/mL.

### DETAILED DESCRIPTION OF THE INVENTION

The present invention discloses the identification of new biomarkers and diagnostic methods for Alzheimer's disease (AD) and related disorders. The invention describes novel use of biomarkers that can be detected in tissues and biological fluids for purposes of diagnosing AD and related disorders. More particularly, this invention relates to new metabolic biomarkers and combinations thereof useful to diagnose AD and related disorders.

### Definitions

Within the context of this invention, "AD related disorders" includes Senile Dementia of AD Type (SDAT), prodromal AD, Mild Cognitive Impairment (MCI), frontotemporal dementia (FTD), vascular dementia and Age-Associated Memory Impairment (AAMI).

It should nevertheless be contemplated that biomarkers of the invention, though particularly devoted to AD and related disorders, might find a use in diagnosing other neurological disorders that share some metabolic features with AD or related disorders, these are, for example, multiple sclerosis, Parkinson's disease or amyotrophic lateral sclerosis.

Within the context of the invention, diagnosing AD and related disorders means identifying or detecting or assessing a risk, presence, subtype, severity or progression of the pathologic condition. More particularly, diagnostic methods of the invention can be used to prognose the development of the disease, to detect the presence of the disease, to identify disease subtype, to monitor the progression of the disease, to qualify AD or related disorders, to assess the responsiveness of a subject to a treatment, to enhance patient stratification step in clinical trials, or to assess the efficacy of a treatment.

The term "biomarker" as used herein refers to a metabolite which can be used to diagnose AD or related disorders in a subject, preferably a human subject, most preferably in a fluid sample from such a subject.

Metabolites are the downstream end products of genome, transcriptome and proteome variability of a biological system. Hence, the term "metabolite" encompasses any substance produced by the metabolism of an organism or by a metabolic process in an organism. For example, metabolites are small molecules as sugars, cholesterol, nucleosides, lipids, amino acids, or even peptides comprising 2, 3, 4, 5, 6, 7 or 8 amino acids.

The term "differentially present", "differential presence" or "differential level" refers to an alteration in the presence, quantity and/or the frequency and/or form of a biomarker in a sample from a diseased subject as compared to a control. The differential presence therefore reflects the presence of a level (or frequency or form) which is different from a "normal" level. The control may be the quantity and/or the frequency and/or the form of the biomarker as determined in a similar sample from a healthy subject, or a reference value (*e.g*., median value, average value), and/or level(s) of the biomarker in a sample from the same subject before disease development and/or at an earlier stage of treatment/disease in the subject, and/or level(s) of the biomarker in a sample from another diseased subject or diseased subject population as control.

"Level" and "quantity" are interchangeable terms.

The terms "alteration" or "deviation" or "difference" in the quantity of a target biomarker may designate an increase or a decrease of the target biomarker quantity in a biological sample from the subject, in comparison with a control sample or reference value. Typically, the term "decrease" in relation to a biomarker level, designates a statistically significant reduction of the concentration or level of the biomarker in a biological sample from the subject. In an embodiment such a decrease is of at least 1% or 3% or 9% in comparison with a control sample or reference or mean value. Decrease may be more substantial, such as a reduction by at least 15% or even more. In a preferred embodiment, decrease may be of 10%, 20%, 30%, 40%, 50%, 60%, 70%, 80%, 90% or 100%. In a more preferred embodiment, decrease may be of 20%, 50% or 60% or even more. Similarly, the term "increase" in relation to the biomarker level, designates a statistically significant augmentation of the concentration or level of the biomarker in a biological sample from the subject. In an embodiment, such an increase is of at least 1% or 3% or 9% in comparison with a control sample or reference or mean value. Increases may be more substantial, such as an increase by at least 15% or even more. In a preferred embodiment, increases may be of 10%, 20%, 30%, 40%, 50%, 60%, 70%, 80%, 90% or 100% (or even more). In a more preferred embodiment, increases may be of 20%, 50% or 60%. Alternatively, an alteration in the frequency of a biomarker can otherwise be observed. Said biomarker(s) can be detected at a higher frequency or at a lower frequency in samples of patients compared to samples of control subjects. A biomarker can be differentially present in terms of quantity, frequency, and/or form, and is indicative of AD or related disorder in the subject. The order of magnitude of said increase or decrease may vary depending on the biomarker, patient, type or stage of disease. The order of variation in the level of biomarker (increase or decrease) as determined and disclosed in the present application is characteristic of the disease.

"Sensitivity", "Specifity" and "AUC" are statistical terms which are commonly used when talking about the predictive power of diagnostic kit. "Sensitivity" reflects the capacity of a test to give a positive result when the hypothesis is verified, and "specificity" the capacity to give a negative result when the hypothesis is rejected. Consequently, in the present invention, a high sensitivity means that the deviation of the biomarker is highly indicative of the disease onset, presence or progression; a high specificity means that the absence of a deviation of the biomarker is highly correlated to the absence of the onset, presence or progression of the disease. The Area under the ROC (Receiver Operating Characteristic) Curve (AUC) is the average sensitivity of the biomarker over the range of specificities. It is often used as a summary statistic representing the overall performance of the biomarker. A biomarker with no predictive value would have an AUC of 0.5. As exemplified in the experimental section, biomarkers which have now been identified by the inventors are characteristic of AD and related disorders. More particularly, though being assayable in the CSF, biomarkers of the invention are metabolites which can also be assayed from body fluids that are more easily obtainable from the subject in comparison with the CSF.

Mining of data on AD and related disorders, new analysis of functional data and experimentations first allowed the inventors to identify pathways implied in the disease. These functional units were then combined and served as a starting point to construct larger functional networks of interacting pathways. Based on these networks, metabolites as candidate biomarkers could be identified and selected by the inventors. Such biomarkers were prioritized for different criteria, including:
- their participation in a signaling pathway associated with onset and development of AD, and
- their participation in the functional network cogently represented by AD-associated pathways.

This led to the identification of metabolites implicated in or interfering within several pathways thereby found to be altered in AD patients.

Further validation studies allowed the selection of valuable metabolite biomarkers that can be used alone, mixed together, or combined with other already known markers to diagnose AD or related disorders. The metabolites are characterized by their monoisotopic mass (table 1) and the m/z value of their dominant ion obtained by mass spectrometry analysis (table 2) as explained in the experimental section. The metabolites listed in table 1 are those for which the identity has been further confirmed using the corresponding internal standard (when commercially available). Atomic mass unit (amu) and m/z are expressed with a 15 ppm standard deviation corresponding to the precision of the measure method. These metabolite biomarkers were further tested to confirm their relevance to AD, as shown in the experimental section.

The metabolites are disclosed in tables 1 and 2 below, with their name, monoisotopoic mass and, when available, the CAS number of some of known salts thereof.

**Table 1**

| **Metabolite name** | **Molecular formula** | **Monoisotopic mass (Da)** | **Illustrative CAS number** |
|---|---|---|---|
| **Azelaic acid** | C9H16O4 | 188.10486 | 123-99-9 |
| **Sebacic acid** | C10H18O4 | 202.12051 | 111-20-6 |
| **Dodecanedioic acid** | C12H22O4 | 230.15181 | 693-23-2 |
| **Tryptophan** | C11H12N2O2 | 204.089878 | 73-22-3 |
| **PFAM (22:1)** | C22H43ON | 338.31848 | n/a |
| **Hippuric acid** | C9H9NO3 | 179.058244 | 495-69-2 |
| **Tyrosine** | C9H11NO3 | 181.073894 | 60-18-4 |
| **Caffeine** | C8H10N4O2 | 194.080376 | 58-08-2 |
| **L-Citrulline** | C6H13N3O3 | 175.095691 | 372-75-8 |
| **Phenylacetylglutamine** | C13H16N2O4 | 264.111007 | 28047-15-6 |
| **Aminoisobutyric acid** | C4H9NO2 | 103.063329 | 62-57-7 (2-) or 144-90-1 (3-) |
| **Aspartate** | C4H7NO4 | 133.037509 | 617-45-8 or 56-84-8 or 1783-96-6 |
| **Aspartyl- Phenylalanine (Asp- Phe)** | C13H16N2O5 | 280.105922 | 13433-09-5 |
| **Glycocholic acid** | C26H43NO6 | 465.309039 | 475-31-0 |
| **Guanosine** | C10H13N5O5 | 283.09167 | 118-00-3 |
| **Inosine** | C10H12N4O5 | 268.080771 | 58-63-9 |
| **L-Threonic acid** | C4H8O5 | 136.037175 | 7306-96-9 |
| **Undecanedioic acid** | C11H20O4 | 216.13616 | 1852-04-6 |

| | | | |
|---|---|---|---|
| n/a: not available | | | |

**Table 2**

| **Corresponding metabolite name** | **M/z ((M+H) or (M-H))** | **Ionization mode** | **Retention time (min)** | **Molecular formula** | **Mono- isotopic mass (Da)** | **Illustrative CAS number** |
|---|---|---|---|---|---|---|
| **1-monopalmitin** | 331,28362 | + | 12,13149 | C19H38O4 | 330.27701 | 73299-28-2 |
| **4-methyl-2- oxovaleric acid** | 129,05427 | - | 4,41515 | C6H10O3 | 130.062995 | 816-66-0 |
| **9,12-dioxo- dodecanoic acid** | 229,14282 | + | 7,166546 | C12H20O4 | 228.13616 | 51551-01-0 |
| **9,12-dioxo- dodecanoic acid** | 227,12787 | - | 7,14630 | | | |
| **Caproic acid** | 115,07516 | - | 4,69762 | C6H12O2 | 116.08373 | 142-62-1 |
| **Isovaleric acid** | 101,05930 | - | 3,44798 | C5H10O2 | 102.06808 | 503-74-2 |
| **Nonenedioic acid** | 187,09584 | + | 5,19255 | C9H14O4 | 186.08921 | n/a |
| **Nonenedioic acid** | 185,08082 | - | 5,17398 | | | |
| **Octadecadienoyl- glycero-3-phosphate** | 433,23620 | - | 10,69926 | C21H41O7P | 436.258993 | n/a |
| **PFAM (20:1)** | 310,30909 | + | 13,01947 | NH39OC20 | 272.013639 | n/a |
| **PFAM (22:2)** | 336,32463 | + | 13,17333 | NH41OC22 | 296.013639 | n/a |
| **Seryl-phenylalanine (Ser-Phe)** | 253,11788 | + | 1,79742 | C12H16N2O4 | 252.111008 | 16875-28-8 |
| | 251,10285 | - | 1,73240 | | | |
| **Sulfobenzylalcohol** | 187,00591 | - | 5,31783 | C7H7O4S | 187.006507 | n/a |
| **Theophylline and/or paraxanthine*** | 181,07141 | + | 3,67416 | C7H8N4O2 | 180.064726 | 58-55-9, 611-59-6 respectively |
| **Valeric acid** | 101,05930 | - | 4,41422 | C5H10O2 | 102.06808 | 109-52-4 |

| | | | | | | |
|---|---|---|---|---|---|---|
| *biomarker that corresponds to theophylline or paraxanthine or a mix thereof; n/a: not available | | | | | | |

The above metabolites represent valuable biomarkers which may be used, alone or in various combinations, for diagnosing AD or related disorders. The ability to detect and monitor levels of these biomarkers provides enhanced diagnostic capability by allowing clinicians to detect risk of developing disease in an early stage, to determine the level of the severity of the disease, to monitor the effects of the therapy by examination of these biomarkers in patient samples, or to sub-classify accurately patient in order, for example, to adapt the treatment or to predict the responsiveness of a patient to a treatment. In comparison to currently existing products, the invention provides several advantages and benefits. The herein described biomarkers provide more rapid, objective and accurate diagnosis of the disease or of its progression than existing diagnostic protocols. For example, neuropsychological tests (as Mini-Mental State Examination, MMSE) are only indicative of an impaired cognition and or dementia; their results can vary as a function of sociocultural factors and are generally taken as only indicative, when considered alone, of the presence or the absence of AD or a related disease. Furthermore, tools such as Amyvid, even if approved by the FDA, can be neither used as a predictive tool nor to appreciate the response to a treatment as stated by this administration.

The inventors have discovered that several primary fatty acid amides (PFAM) represent valuable biomarkers. Preferred PFAM are PFAM (22:1), PFAM (20:1), and PFAM (22:2). In this regard, PFAM of this invention have the following formula:

NH₂-CO-R

with R being either i) in the case of PFAM (20:1), an alkene of 19 carbon atoms with one cis or trans double bond or ii) in the case of PFAM (22:1), an alkene of 21 carbon atoms with one cis or trans double bond or iii) in the case of PFAM (22:2), an alkene of 21 carbon atoms with two double bonds that are independently cis or trans.

Consequently, in the context of the invention, PFAM (20:1) designates one single isomer or a mix of PFAM (20:1) isomers, PFAM (22:1) designates one single isomer or a mix of PFAM (22:1) isomers, and PFAM (22:2) designates one single isomer or a mix of PFAM (22:2) isomers.

In the context of the invention, "C₇H₈N₄O₂" designates either theophylline alone, or paraxanthine alone, or a mix thereof.

The invention may be further used to predict the onset of AD and related disorders in advance of the appearance of any symptom conventionally used in the diagnostic of the disease. Thus the invention may be used in the testing and monitoring of individuals believed to be at risk of developing AD or a related disorder e.g. individuals with a family history of the disease, in order to enable early intervention to prevent onset or development of the symptoms. Such testing and monitoring may be used to identify or predict the development of AD and related disorders months or years in advance of the onset of the disease.

In other aspects, methods of the present invention further comprise the step of managing the individual treatment. For example, managing treatment comprises administering a matched drug or drug combination to slow, to halt or to reverse the progression of the disease. In another aspect of the invention, the method further comprises measuring the biomarker level after the treatment has begun, monitoring the progression of the disease, the response to the treatment or even the efficiency of the said selected treatment. In a particular embodiment monitoring the response to the treatment comprises determining the differential presence, in a biological fluid sample from the subject, of one or more of the above biomarkers, after administration of said treatment or at different point of times during the course of the treatment; a significant differential presence (whatever the order of variation) compared to the reference value being indicative of a response to the treatment.

As far as chronic diseases are concerned, in a particular embodiment, the monitoring of the response to the treatment comprises determining the differential presence, in a biological fluid from the subject, of one or more of the above biomarkers at different points of time during the course of the treatment.

In another particular embodiment, the monitoring of the disease progression comprises determining the differential presence, in a biological fluid from the subject, of one or more of the above biomarkers at different points of time during the course of the treatment.

In another particular embodiment, monitoring the efficiency of the treatment comprises determining the differential presence, in a biological fluid sample from the subject, of one or more of the above biomarkers, after administration of said treatment or at different point of times during the course of the treatment; a correction of such differential presence (i.e. an evolution toward a "normal state" level) during treatment being indicative of an effective treatment.

Also described herein is a method for diagnosing AD or related disorders, which comprises detecting or measuring the differential presence of at least one biomarker, selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe or sulfobenzylalcohol, in a mammal-derived sample, more preferably in a human-derived sample, such as differential presence being indicative of the disease.

More particularly, an object of this invention is a method for diagnosing AD or related disorder in a mammal, the method comprising determining the differential presence of sebacic acid in a sample from the subject, such a differential presence being indicative of the disease.

The sample may be, or may derive from, any metabolite-containing sample obtained from a subject such as a biological fluid, a gas, exhaled breath and/or aerosols, a biopsy, tissue extract, stool, etc. Preferably the sample is or derives from a biological fluid, more preferably from blood (or plasma and/or serum derived therefrom), urine, CSF, etc.

In this regard, disclosed herein is the method comprising determining the differential presence of at least one biomarker, selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe or sulfobenzylalcohol in a biological fluid from the subject, such a differential presence being indicative of the disease.

Also disclosed herein is the method comprising determining the differential presence of at least one biomarker, selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe or sulfobenzylalcohol in blood, plasma and/or serum from the subject, such a differential presence being indicative of the disease.

Also disclosed herein is a method for diagnosing AD or related disorder in a mammal, the method comprising determining the differential presence of at least one biomarker, selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), valeric acid, in blood, plasma and/or serum from the subject, such a differential presence being indicative of the disease.

Also disclosed herein is a method for diagnosing AD or related disorder in a mammal, the method comprising determining the differential presence of at least one biomarker, selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe or sulfobenzylalcohol in the exhaled breath and/or aerosols from the subject, such a differential presence being indicative of the disease.

For example, diagnosing AD and related disorders, comprises the determination of the differential presence, in a biological fluid sample of the mammal, of one or more metabolite(s) selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe or sulfobenzylalcohol.

Also described herein is an *in vitro* method for diagnosing AD or related disorders, the method comprising determining the differential presence of at least one biomarker, selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe or sulfobenzylalcohol, in a biological fluid sample from the subject, wherein said differential presence is indicative of the presence, risk, progression or severity of said disease.

For example, disclosed is diagnosing AD or related disorders comprising measuring, in a biological fluid sample of the mammal, an increase of at least one biomarker selected from aspartate, Asp-Phe, azelaic acid, dodecanedioic acid, phenylacetylglutamine, sebacic acid, undecanedioic acid, 1-monopalmitin, 9,12-dioxo-dodecanoic acid, caproic acid, iso-valeric acid, nonenedioic acid, octadecadienoyl-glycero-3-phosphate, or sulfobenzylalcohol, and/or a decrease of at least one biomarker selected from caffeine, glycocholic acid, guanosine, hippuric acid, inosine, L-citrulline, L-threonic acid, PFAM (22:1), tryptophan, tyrosine, 4-methyl-2-oxovaleric acid, PFAM (20:1), PFAM (22:2), Ser-Phe, C₇H₈N₄O₂ (theophylline and/or paraxanthine), or valeric acid.

Also disclosed herein is diagnosing AD or related disorders comprising measuring, in a biological fluid sample of the mammal, an increase of at least one biomarker selected from azelaic acid, dodecanedioic acid, phenylacetylglutamine, sebacic acid, caproic acid, iso-valeric acid, and/or a decrease of at least one biomarker selected from caffeine, hippuric acid, L-citrulline, PFAM (22:1), tryptophan, tyrosine, 4-methyl-2-oxovaleric acid, PFAM (20:1), PFAM (22:2), C₇H₈N₄O₂ (theophylline and/or paraxanthine), or valeric acid.

In a particular embodiment, the invention relates to an in vitro method for diagnosing a neurological disease selected from Alzheimer's disease (AD), senile dementia of AD type, prodromal AD, mild cognitive impairment, age associated memory impairment, vascular dementia or frontotemporal dementia, said method comprising the following steps:
- collecting blood, serum or plasma sample from a subject suffering from, or suspected to suffer from, or at risk of suffering from said disease,
- treating samples for their further analysis by LC/MS and/or GC/MS,
- measuring by LC/MS and/or GC/MS an increase, as compared to a control value, of sebacic acid and/or a decrease, as compared to a control value, of at least one biomarker selected from caffeine, glycocholic acid, guanosine, hippuric acid, inosine, L-citrulline, L-threonic acid, PFAM (22:1), tryptophan, tyrosine, 4-methyl-2-oxovaleric acid, PFAM (20:1), PFAM (22:2), Ser-Phe, C7H8N4O2 (theophylline and/or paraxanthine), or valeric acid.
- deducing from the preceding step the presence, risk, subtype, progression or severity of said disease.

In an even more preferred embodiment, methods for diagnosing AD or related disorders of the present invention comprise determining the differential presence of a combination of several biomarkers of the present invention, named set of biomarkers. A set contains preferably 2, 3, 4 or 5 (or even more) biomarkers from the above listed biomarkers, which may be determined simultaneously or sequentially in the sample.

Disclosed herein is a set of biomarkers constituted of at least two metabolites selected from the group comprising azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe or sulfobenzylalcohol.

Also disclosed herein is a set of biomarkers constituted of at least two metabolites selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), or valeric acid.

Also disclosed herein is the set of biomarkers constituted of at least three metabolites selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, PFAM (20:1), PFAM (22:1), PFAM (22:2), phenylacetylglutamine, C₇H₈N₄O₂ (theophylline and/or paraxanthine), or valeric acid.

Also disclosed herein is a set of biomarkers containing at least one dipeptide selected from Ser-Phe and Asp-Phe.

Also disclosed herein is a set of biomarkers containing at least one carboxylic acid selected from azelaic acid, sebacic acid, dodecanedioic acid, hippuric acid, valeric acid, iso-valeric acid, 4-methyl-2-oxovaleric acid, caproic acid, L-citrulline, phenylacetylglutamine, aminoisobutyric acid, aspartate, L-threonic acid, undecanedioic acid, 9,12-dioxo-dodecanoic acid, nonenedioic acid, octadecadienoyl-glycero-3-phosphate, or sulfobenzylalcohol, more preferably the carboxylic acid is a-dicarboxylic acid selected from azelaic acid, dodecanedioic acid, sebacic acid, undecanedioic acid, nonenedioic acid.

Also disclosed herein is a set of biomarkers comprising at least one dicarboxylic acid selected from azelaic acid, dodecanedioic acid, sebacic acid, undecanedioic acid, nonenedioic acid, even more preferably said at least one dicarboxylic acid is selected from sebacic acid or azelaic acid.

Also disclosed herein is a set of biomarkers containing at least one PFAM selected from PFAM (20:1), PFAM (22:1) and PFAM (22:2).

Also disclosed herein is the set of biomarkers constituted of at least one PFAM selected from PFAM (20:1), PFAM (22:1) or PFAM (22:2) used in combination with at least one metabolite selected from azelaic acid, dodecanedioic acid, hippuric acid, sebacic acid, tryptophan, tyrosine, 4-methyl-2-oxovaleric acid, caffeine, caproic acid, iso-valeric acid, L-citrulline, phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe and sulfobenzylalcohol.

Also disclosed herein is the set of biomarkers comprising at least two biomarkers selected from sebacic acid, dodecanedioic acid and tryptophan.

In an embodiment, the set of biomarkers comprises sebacic acid, dodecanedioic acid and tryptophan.

In a particular embodiment, sebacic acid concentration is increased from 10 to 90%, preferably from 30% to 70%, and more preferably of 50%, in diseased subjects as compared to a concentration level in a control sample or in a reference situation.

In a particular embodiment, dodecanedioic acid concentration is increased from 10 to 90%, preferably from 40% to 80%, and more preferably of 60%, in diseased subjects as compared to a concentration level in a control sample or in a reference situation.

In a particular embodiment, tryptophan concentration is decreased from 10 to 90%, preferably from 10% to 50%, and more preferably of 20%, in diseased subjects as compared to a concentration level in a control sample or in a reference situation.

In another embodiment, the set of biomarkers comprises sebacic acid, dodecanedioic acid and tryptophan, in combination with at least one metabolite selected from PFAM (20:1), PFAM (22:1), PFAM (22:2), azelaic acid, hippuric acid, tyrosine, 4-methyl-2-oxovaleric acid, caffeine, caproic acid, iso-valeric acid, L-citrulline, phenylacetylglutamine, C₇H₈N₄O₂ (theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxo-dodecanoic acid, nonenedioic acid, octadecadienoyl-glycero-3-phosphate, Ser-Phe and sulfobenzylalcohol.

Also disclosed herein is the set of biomarkers constituted of at least two compounds selected from 1-monopalmitin, dodecanedioic acid, hippuric acid, iso-valeric acid, sebacic acid, tryptophan, tyrosine and undecanedioic acid.
Also disclosed herein are sets of biomarkers selected from sets comprising:
- PFAM (20:1) and PFAM (22:1),
- PFAM (20:1) and PFAM (22:2),
- PFAM (22:1) and PFAM (22:2),
- PFAM (20:1) and PFAM (22:1) and PFAM (22:2),
- Asp-Phe and Ser-Phe,
- Asp-Phe and tryptophan and caproic acid,
- Asp-Phe and azelaic acid and L-threonic acid,
- Asp-Phe and nonenedioic acid and tryptophan and L-threonic acid,
- Asp-Phe and C₇H₈N₄O₂ (theophylline and/or paraxanthine) and L-threonic acid and sebacic acid,
- Asp-Phe and Ser-Phe and caffeine,
- Asp-Phe and dodecanedioic acid and Ser-Phe,
- Asp-Phe and guanosine and Ser-Phe,
- Asp-Phe and hippuric acid and Ser-Phe,
- Asp-Phe and 4-methyl-2-oxovaleric acid and Ser-Phe,
- Asp-Phe and Ser-Phe and octadecadienoyl-glycero-3-phosphate,
- Asp-Phe and Ser-Phe and 9,12-dioxo-dodecanoic acid,
- Asp-Phe and Ser-Phe and phenylacetylglutamine,
- Asp-Phe and valeric acid and Ser-Phe,
- Ser-Phe and caproic acid and undecanedioic acid,
- Ser-Phe and L-citrulline and inosine and aspartate,
- Ser-Phe and tyrosine and 1-monopalmitin and aspartate,
- Ser-Phe and nonenedioic acid and undecanedioic acid and sulfobenzylalcohol,
- L-citrulline and iso-valeric acid and aspartate,
- L-citrulline and tryptophan and aspartate and L-threonic acid,
- L-citrulline and undecanedioic acid and aspartate and sulfobenzylalcohol,
- L-citrulline and azelaic acid and aspartate and glycocholic acid,
- L-citrulline and C₇H₈N₄O₂ (theophylline and/or paraxanthine) and azelaic acid,
- L-citrulline and azelaic acid and valeric acid and phenylacetylglutamine,
- L-citrulline and hippuric acid and sebacic acid and dodecanedioic acid and tryptophan,
- L-citrulline and azelaic acid and tryptophan and 4-methyl-2-oxovaleric acid,
- L-citrulline and azelaic acid and tryptophan and iso-valeric acid and phenylacetylglutamine,
- L-citrulline and tyrosine and azelaic acid and tryptophan and iso-valeric acid,
- Azelaic acid and dodecanedioic acid,
- Azelaic acid and sebacic acid,
- Azelaic acid and sebacic acid and dodecanedioic acid,
- Azelaic acid and undecanedioic acid,
- Azelaic acid and undecanedioic acid and dodecanedioic acid,
- Azelaic acid and undecanedioic acid and sebacic acid,
- Azelaic acid and undecanedioic acid and sebacic acid and dodecanedioic acid,
- Nonenedioic acid and azelaic acid,
- Nonenedioic acid and azelaic acid and dodecanedioic acid,
- Nonenedioic acid and azelaic acid and sebacic acid,
- Nonenedioic acid and azelaic acid and sebacic acid and dodecanedioic acid,
- Nonenedioic acid and azelaic acid and undecanedioic acid,
- Nonenedioic acid and azelaic acid and undecanedioic acid and dodecanedioic acid,
- Nonenedioic acid and azelaic acid and undecanedioic acid and sebacic acid,
- Nonenedioic acid and azelaic acid and undecanedioic acid and sebacic acid and dodecanedioic acid,
- Nonenedioic acid and dodecanedioic acid,
- Nonenedioic acid and sebacic acid,
- Nonenedioic acid and sebacic acid and dodecanedioic acid,
- Nonenedioic acid and undecanedioic acid,
- Nonenedioic acid and undecanedioic acid and dodecanedioic acid,
- Nonenedioic acid and undecanedioic acid and sebacic acid,
- Nonenedioic acid and undecanedioic acid and sebacic acid and dodecanedioic acid,
- Sebacic acid and dodecanedioic acid,
- Undecanedioic acid and dodecanedioic acid,
- Undecanedioic acid and sebacic acid,
- Undecanedioic acid and sebacic acid and dodecanedioic acid
- Sebacic acid and tyrosine,
- Hippuric acid and sebacic acid and tyrosine,
- Sebacic acid and tyrosine and undecanedioic acid,
- Sebacic acid and tryptophan and tyrosine,
- Sebacic acid and tryptophan,
- Hippuric acid and sebacic acid and tyrosine and undecanedioic acid,
- Dodecanedioic acid and sebacic acid and tyrosine and undecanedioic acid,
- Dodecanedioic acid and sebacic acid and tyrosine,
- Sebacic acid and tryptophan and undecanedioic acid,
- Dodecanedioic acid and sebacic acid and tryptophan and tyrosine,
- Hippuric acid and sebacic acid and tryptophan and tyrosine,
- Dodecanedioic acid and sebacic acid and tryptophan,
- Dodecanedioic acid and hippuric acid and sebacic acid and tyrosine and undecanedioic acid,
- Hippuric acid and sebacic acid,
- Dodecanedioic acid and hippuric acid and sebacic acid and tyrosine,
- Sebacic acid and tryptophan and tyrosine and undecanedioic acid,
- Hippuric acid and sebacic acid and tryptophan and undecanedioic acid,
- Hippuric acid and sebacic acid and tryptophan,
- Dodecanedioic acid and sebacic acid and tryptophan and undecanedioic acid,
- Hippuric acid and sebacic acid and tryptophan and tyrosine and undecanedioic acid,
- Dodecanedioic acid and sebacic acid and tryptophan and tyrosine and undecanedioic acid,
- Dodecanedioic acid and hippuric acid and sebacic acid and tryptophan and tyrosine,
- Dodecanedioic acid and hippuric acid and sebacic acid,
- Hippuric acid and sebacic acid and undecanedioic acid,
- Dodecanedioic acid and hippuric acid and sebacic acid and tryptophan,
- Dodecanedioic acid and hippuric acid and sebacic acid and undecanedioic acid,
- Dodecanedioic acid and hippuric acid and sebacic acid and tryptophan and tyrosine and undecanedioic acid,
- Dodecanedioic acid and sebacic acid,
- Dodecanedioic acid and hippuric acid and tryptophan,
- Dodecanedioic acid and hippuric acid,
- Dodecanedioic acid and hippuric acid and sebacic acid and tryptophan and undecanedioic acid,
- Dodecanedioic acid and tyrosine,
- Dodecanedioic acid and tryptophan,
- Dodecanedioic acid and hippuric acid and tyrosine,
- Dodecanedioic acid and hippuric acid and tryptophan and undecanedioic acid,
- Hippuric acid and tryptophan,
- Dodecanedioic acid and hippuric acid and undecanedioic acid,
- Dodecanedioic acid and hippuric acid and tryptophan and tyrosine,
- Dodecanedioic acid and hippuric acid and tyrosine and undecanedioic acid,
- Dodecanedioic acid and tryptophan and tyrosine,
- Dodecanedioic acid and tryptophan and undecanedioic acid,
- Dodecanedioic acid and tyrosine and undecanedioic acid,
- Hippuric acid and tyrosine and undecanedioic acid,
- Hippuric acid and tryptophan and tyrosine,
- Hippuric acid and tryptophan and undecanedioic acid,
- Hippuric acid and undecanedioic acid,
- Hippuric acid and tryptophan and tyrosine and undecanedioic acid,
- Hippuric acid and tyrosine,
- Dodecanedioic acid and tryptophan and tyrosine and undecanedioic acid,
- Dodecanedioic acid and hippuric acid and tryptophan and tyrosine and undecanedioic acid,
- Tyrosine and undecanedioic acid,
- Tryptophan and undecanedioic acid,
- Tryptophan and tyrosine and undecanedioic acid, or
- Tryptophan and tyrosine.

In an embodiment of the invention, preferred sets of biomarkers are selected from:
- Asp-Phe and C₇H₈N₄O₂ (theophylline and/or paraxanthine) and L-threonic acid and sebacic acid,
- L-citrulline and hippuric acid and sebacic acid and dodecanedioic acid and tryptophan,
- Sebacic acid and tryptophan and tyrosine,
- Sebacic acid and tryptophan,
- Sebacic acid and tryptophan and undecanedioic acid,
- Hippuric acid and sebacic acid and tryptophan and tyrosine,
- Dodecanedioic acid and sebacic acid and tryptophan,
- Hippuric acid and sebacic acid,
- Sebacic acid and tryptophan and tyrosine and undecanedioic acid,
- Hippuric acid and sebacic acid and tryptophan and undecanedioic acid,
- Hippuric acid and sebacic acid and tryptophan,
- Dodecanedioic acid and sebacic acid and tryptophan and undecanedioic acid,
- Hippuric acid and sebacic acid and tryptophan and tyrosine and undecanedioic acid,
- Dodecanedioic acid and sebacic acid and tryptophan and tyrosine and undecanedioic acid,
- Dodecanedioic acid and hippuric acid and sebacic acid and tryptophan and tyrosine,
- Dodecanedioic acid and hippuric acid and sebacic acid,
- Hippuric acid and sebacic acid and undecanedioic acid,
- Dodecanedioic acid and hippuric acid and sebacic acid and tryptophan,
- Dodecanedioic acid and hippuric acid and sebacic acid and undecanedioic acid,
- Dodecanedioic acid and hippuric acid and sebacic acid and tryptophan and tyrosine and undecanedioic acid,
- Dodecanedioic acid and sebacic acid, or
- Dodecanedioic acid and hippuric acid and sebacic acid and tryptophan and undecanedioic acid;

In a particular embodiment of the invention, a sets of biomarkers is:
- C7H8N4O2 (theophylline and/or paraxanthine) and Asp-Phe and L-threonic acid and sebacic acid,

Also disclosed herein is diagnosing AD and related disorders, comprising the identification, within LC/MS or GC/MS mass profile from sample of the mammal, of a metabolite mass profile determined as specific for AD or a related disorder said profile being constituted by 2, 3, 4 or 5 mass peaks corresponding to the dominant ions of the metabolites identified in tables 1 and 2.

In a particular embodiment, any of the above biomarkers or their combinations are used in a method of diagnosing AD or related disorders, in conjunction with at least one additional diagnostic test or biomarker for AD or related disorders, selected preferably from nucleic acids, proteins, metabolites, neurophysiological (e.g. electroencephalography), genetic, brain imaging, clinical and cognitive test or biomarker. Such diagnostic test or biomarker can be done or measured concomitantly, before, or after the measure of biomarkers of the invention. Said additional diagnostic biomarkers can be detected in any sample convenient for the assay.

Said additional protein biomarker, which can be used for diagnosing AD or related disorders, can be selected from proteins listed in WO2011/012672. Other candidates as proteinaceous biomarkers known in the art as an aid in diagnosing AD are Aβ₄₂, Tau or P-Tau₁₈₁, which can be dosed from the LCR. A decreased in Aβ₄₂, and an increase of Tau and P-Tau₁₈₁ are noticed in the LCR of AD patients. When talking about plasmatic biomarkers, the usefulness of Aβ peptides is at least controversial [17], but Aβ₄₂/Aβ₄₀ ratio seems to be of some use as a low Aβ₄₂/Aβ₄₀ plasmatic ratio has been associated with the risk of a more rapid cognitive decline [17].

Consequently, in an embodiment, any of the biomarkers of the invention or their combinations are used in a method of diagnosing AD or related disorders, in conjunction with the measure of the determination of Aβ₄₂, Tau and/or P-Tau₁₈₁ in the LCR.

In another embodiment any of the biomarkers of the invention or their combinations are used in a method of diagnosing AD or related disorders or the risk of a rapid cognitive decline, in conjunction with the measure of plasmatic Aβ₄₂/Aβ₄₀ ratio.

Brain imaging tests that can be implemented in conjunction with any of the biomarkers of the invention can be for example:
- detection and quantitation tests of Aβ deposition and/or fibrillar Aβ burden in brain, or of pattern of deposition thereof, by imaging methods as positron emission tomography, which can be indicative of AD or of AD evolution,
- morphologic brain imaging, for instance measure of the volume of the hippocampus, which can be indicative of AD or of AD evolution.

In a more particular embodiment, biomarkers of the invention are used to diagnose AD or a related disorder in patient(s) identified as being at risk of developing AD or suspected of suffering from prodromal AD. For instance such patient(s) can have been diagnosed bearing ApoE ε4 allele of ApoE.

Biomarkers of the invention can also be used in addition of any cognitive test used to assess the cognitive status of a patient. Such tests are, for example, Mini-Mental State Examination (MMSE), Modified Mini-Mental State Examination (3MS), Abbreviated Mental Test Score (AMTS), Dementia questionnaire for persons with Mental Retardation (DMR), Cognitive Abilities Screening Instrument (CASI), Trail-making test, Clock drawing test, Alzheimer's disease assessment scale - Cognition (ADAS-Cog), General Practitioner Assessment of Cognition (GPCOG), Montreal Cognitive Assessment (MoCA), or Rowland Universal Dementia Assessment Scale (RUDAS).

In a preferred embodiment, any of the biomarkers of the invention is used in conjunction with MMSE.

In another preferred embodiment, biomarkers of the invention are used to diagnose AD or a related disorder in patient(s) identified as being at risk of developing AD or suspected of suffering from prodromal AD because of the result they obtained in the MMSE. As pointed out above, the MMSE scores are affected by the age and the cultural level of the subject. Thus these scores must be corrected in function of these criteria before their interpretation. As an indicative basis, according to the Consortium to Establish a Registry for Alzheimer's Disease (CERAD), a score comprised between 19 and 24 is associated with a weak dementia, between 10 and 18 with a moderate dementia and finally, a score under 10 corresponds to a severe dementia.

Another aspect of the disclosure relates to the use of one or more biomarker(s) selected from biomarkers disclosed herein in a method of AD diagnosis in a mammalian subject.

The method of the invention is applicable to any biological sample of the mammal to be tested. Examples of such samples include blood, plasma, serum, saliva, urine, ascites, sputum, aerosols, sweat or the like. Level of metabolites derived therefrom can also be measured from tissue biopsies or feces. The sample can be obtained by any technique known *per se* in the art, for example by collection using e.g., non-invasive techniques, or from collections or banks of samples, etc. The sample can in addition be pretreated to facilitate the accessibility of the target biomarker, to allow the dosage of said biomarker by a dedicated method (e.g. derivatization of amino acids to allow their subsequent dosage by spectrophotometry), or to enrich for the target biomarker, for example by lysis (mechanical, chemical, enzymatic, etc.), purification, extraction, centrifugation, separation, precipitation, etc. Serum preparation from blood can be performed as exemplified in experimental section. Several other sample preparations can be used such as liquid-liquid extraction, protein precipitation and solid-phase extraction [18]. In a preferred embodiment, levels of biomarkers of the invention are determined from blood, plasma, serum, saliva, or urine sample(s).

In another embodiment, biomarker(s) may be quantified from different samples from the same mammal.

The invention is applicable to any mammal, preferably to a human.

In an embodiment, said human is not yet suffering from a significant cognitive impairment when compared with people of same age and cultural level.

In another embodiment, said human presents Aβ aggregates deposition or a fibrillar Aβ burden in brain, associated or not with a cognitive impairment.

It is known that patients with Down's syndrome exhibit an extremely high incidence of early onset of AD [19]. Consequently, in another embodiment, said human is suffering from Down's syndrome.

The levels of said biomarker(s) may be determined by any method known *per se* in the art, such as, without limitation, immunological methods, biochemical methods, chromatographic methods, enzymatic methods, cell based assays, *in vitro* tests, LC/MS, GC/MS etc. Such assays are routine and well known in the art. The levels of biomarker(s) determined may be compared to a reference value, a control, or a mean value, wherein a deviation from said value is indicative of the presence, risk, progression and/or severity of AD or related disorders. The deviation should typically be superior to 1%, preferably superior to 3%, more preferably superior to 9%, even more preferably superior to 15%. In other embodiments, deviation may be of 10%, 20%, 30%, 40%, 50%, 60%, 70%, 80%, 90% or 100%.

In another embodiment, differential presence of other metabolites related to the same metabolic pathways than the biomarkers of the invention is quantified.

Also described herein is a kit comprising a solid support comprising at least one capture agent attached thereto, wherein said at least one capture agent binds or reacts with one biomarker of the present invention. Typically, the kit may comprise several distinct capture agents which bind to a distinct biomarker. The at least one binding agent is preferably selective for a biomarker, such as an antibody or a derivative thereof, an aptamer, etc.

For example, the kit comprises a solid support comprising at least one capture agent attached thereto (for instance an antibody or an aptamer), wherein the capture agent binds or reacts with one biomarker from the biomarkers disclosed herein. In this regard, the kit comprises at least one compound binding to or reacting with at least one biomarker selected from the biomarkers disclosed herein for the diagnostic, prognostic and/or for assessing the efficacy of a treatment or following the evolution of AD or related disorders.

In addition to LC/MS method for assaying biomarkers of the invention, other assays exist as discussed below in an illustrative way.

### Amino acids (or derivative thereof) quantification

### HPLC-spectrophotometry whole amino acids profile.

Amino acids blood tests are well known in the art. They are, for example, commonly used to determine aminogram of young children in order to diagnose aminoacidopathies.

HPLC/spectrophotometry methods are the most commonly used methods for assaying whole amino acids (or their derivatives) at once from biological fluids. They are more often automatized. Amino acids need to be derivatized to be detectable by absorbance spectrophotometry. Derivatization can be performed before or after HPLC amino acids separation.

Derivatization consists in the covalently linking of amino acids to a chromophoric moiety thereby rendering modified amino acids easily detectable by UV, visible or fluorometric spectrophotometry. Derivatization can be performed, for example, with Phenyl-Thio-Cyanate (PTC, UV spectrophotometry), Ortho-PhtAldehyde, (OPA; UV or fluorometric spectro-photometry), DimethylAmino-1-NaphtaleneSulfonYL (DANSYL; visible spectrophotometry), or 9-FluorenylMethOxyCarbonyl (FMOC; fluorometric spectrophotometry).

Protocol for amino acids quantization using OPA derivatization is extensively described in Babu *et al.* [20].

Commercial kits are also sold for performing HPLC assays to measure amino acids quantity in human fluids as for example "Phenylalanine, Tyrosine & Tryptophan HPLC Assay" from Eagle biosciences (Catalog Number: PNL31-H100).

### Kits dedicated to the quantification of specific amino acids

Amino acids biomarkers can also be specifically quantified from biological samples using off the shelf dedicated detection and quantification kits.

Aspartic acid can be assayed using, for example, "Aspartate assay kit" (Biovision, ref K552-100): an enzymatic colorimetric assay based of the enzymatic conversion of aspartate in pyruvate. L-tryptophan can be measured using "Bridge-It® L-Tryptophan Fluorescence Assay" (Mediomics) which is based on the activity of tryptophan repressor protein and can detect tryptophan for instance in human urine or serum.

### Fatty acid detection and quantitation

Fatty acids used in the invention and related compounds (*i*.*e*. dodecanedioic acid; sebacic acid; azelaic acid, caproic acid, undecanedioic acid, 9,12-dioxo-dodecanoic acid, nonenedioic acid, octadecadienoyl-glycero-3-phosphate) can also be identified by HPLC (reviewed by Lima and Abdalla, 2002, and Chen and Chuang, 2002) [21,22] or GC methods (see in Bondia-Pons *et al.* in 2004 [23] for example) well known by the man of the art. These methods usually need sample preparation steps as lipids extraction, purification and derivatization; they can be coupled or not to different detection and quantification methods, depending on the derivatization method that has been used. Reference compounds can easily be found to allow correct identification of the fatty acids which are searched for.

### Immunological and aptamers based methods

Immunological methods are methods that use an antibody to specifically bind an antigen (*e.g*. a biomarker, fragments and derivatives thereof.). The immunological method is used, in particular, to isolate, target, and/or quantify the antigen. For example, immunological methods include but are not limited to competitive and non-competitive assay systems using techniques such as western blots, radioimmunoassays, ELISA, "sandwich" immunoassays, immunoprecipitation assays, immunodiffusion assays, fluorescent immunoassays.

Antibody refers to a polypeptide ligand substantially encoded by an immunoglobulin gene or immunoglobulin genes, or fragments thereof, which specifically binds and recognizes an epitope (*e.g.* an antigen). The term "antibody", as used herein, also includes antibody fragments either produced by the modification of whole antibodies or those synthetized *de novo* using recombinant DNA methodologies. It also includes polyclonal antibodies, monoclonal antibodies, chimeric antibodies, humanized antibodies or single chain antibodies.

Detection methods for assaying metabolites used in the invention could use an aptamer that specifically binds to the searched metabolites. Aptamers are synthetic ssDNA or RNA molecules that recognize a ligand with a high specificity and affinity; they can represent a valuable alternative to antibodies in the case of metabolites with no or a low immunogenicity. They can be used for assaying metabolites of any kind, and their specificity allows the differentiation of closely related molecules. They can be easily synthetized by selex technique and variations thereof which are well known in the art [24] or chosen from a commercial library as for instance that of Aptagen (www.aptagen.com). Detection or quantification is performed somewhat in the same way that for well-known immunological methods or with dedicated methods[25].

Further aspects and advantages of this invention will be disclosed in the following experimental section, which shall be considered as illustrative only.

### EXAMPLES

### A) Identification of biomarkers of AD from human samples

### 1. Sample preparation

### 1.1. Human serum samples

Samples and clinical data were handled in accordance with the highest ethical standards and in strictest compliance with all applicable rules and regulations including the recommendations of the Council of the Human Genome Organization (HUGO) Ethical, Legal, and Social Issues Committee (HUGO-ELSI, 1998); with the United Nations Educational, Scientific, and Cultural Organization's (UNESCO) Universal Declaration on the Human Genome and Human Rights (1997); and with recommendations guiding physicians in biomedical research involving human subjects adopted by the 18th World Medical Assembly, Helsinki, Finland, 1964 and later revisions. All samples were collected under U.S. IRB approved clinical protocols.

Two sets of human serum samples were collected.

The first set was used to identify biomarkers from experimental rounds of LC/MS identification, validation and characterization of mass peaks obtained from LC/MS analyses in the frame of the pathways identified as altered as explained above. The second, despite some differences in selection criteria of samples, confirmed the usefulness of the biomarkers of the invention in discriminating AD patients from controls

### 1.1.1. First set of samples

Serum samples from 50 AD subjects, 3 mild cognitive impaired (MCI) subjects and 48 non-demented elderly controls (CTRL) were obtained from PrecisionMed (San Diego, CA, USA). Diagnosis of AD was based on medical evaluation and neuropsychiatric testings.

The selection criteria of the first set of samples are the following:
- homogenous age groups (> 65 years old),
- fasting donors (samples collected on the morning),
- homogenous sampling date, specimen collection center, sampling protocol between AD, MCI patients and controls,
- adjusted proportion of men and women.

Data about this sample collection are summarized in table 3 below.

**Table 3**

| **Data** | **AD (n = 50)** | **MCI (n = 3)** | **CTRL (n = 48)** | **P-value** |
|---|---|---|---|---|
| **Gender (F/M)** | 25/25 | 1/2 | 24/24 | 0.851 |
| **BMI (kg/m²)** | 27.5±6.1 | 24.9±2.5 | 29.7±5.9 | 0.124 |
| **MMSE** | 17.7±4.7 | 21.3±3.1 | 29.3±1.4 | 8.41E-28 |

| | | | | |
|---|---|---|---|---|
| BMI: Body Mass Index | | | | |

### 1.1.2. Second set of samples

Serum samples from 42 AD subjects and 33 non-demented elderly controls (CTRL) were obtained from ABS Inc. (Wilmington, DE, USA). Diagnosis of AD was based on medical evaluation and neuropsychiatric testings.

The selection criteria of the second set of samples are the following:
- homogenous age groups (> 65 years old),
- homogenous sampling date, specimen collection center, sampling protocol between AD patients and controls,

The AD subjects of this second set were all administrated with cerebrolysin. Data about this sample collection are summarized in table 4 below.

**Table 4**

| **Data** | **AD (n = 42)** | **CTRL (n = 33)** | **P-value** |
|---|---|---|---|
| **Gender (F/M)** | 29/13 | 15/18 | 0.0682 |
| **BMI (kg/m²)** | 24.4 | 25.9 | 0.026 |
| **MMSE** | 10.1 | n/a | n/a |

| | | | |
|---|---|---|---|
| BMI: Body Mass Index; n/a: not available | | | |

Serum separating tubes were gently inverted 5 times to mix the clot activator with blood; blood was then allowed to clot for at least 30 min at room temperature in a vertical position. Tubes were then centrifuged at 1300-1500 g at room temperature within maximum 2 hours of collection, for approximately 10 min.

Aliquoted sera were then freeze immediately at -80°C.

### 1.2. Human plasma samples

Plasmas from 28 healthy control subjects and 27 AD patients have been collected. AD samples came from Department of Neurology, Memory Research Resources Center (Montpellier University Hospital Gui de Chauliac, France) and plasma samples of age-matched controls were collected by Institut de Santé Publique d'Epidémiologie et de Développement (ISPED, University of Bordeaux, France).

These human plasma biomarkers are also found in human sera.

### 1.3. Mouse samples from Tg2576, a mouse model for AD

Eleven female Tg(HuAPP695.K670N-M671L)2576 (human APP695 Swedish= Tg2576) and twelve wild-type littermates (WT) were used for performing the study in animal. As stated by Hsiao [26], this transgenic mouse shows behavioral, biochemical and pathological features which can be considered similar to that observed in AD humans.

In this model, onset of symptoms begins at 9-10 months. At 17 months, after cervical dislocation, mice were subjected to cardiac puncture and blood samples of 1 mL were collected into heparinized pre-cooled tubes. The whole blood was immediately centrifuged at 3000 g for 15 min at 4°C. Plasma was then carefully removed from the pellet after centrifugation to avoid any contamination by red blood cells, and aliquots of approximately 100 µL were stored into 1.5 mL polypropylene tubes at -80°C. Sample treatment and LC/MS analysis was then carried as explained herein after.

### 1.4. Sample processing

After thawing of the deep-frozen samples at room temperature, a step of protein precipitation with methanol (MeOH) was performed. Four volumes of MeOH were added to 50 µL of each serum. Then, the mixed solutions are sonicated, vortexed and centrifuged (during 20 min, at 8000 g and 4°C) before recovery of the supernatant (about 350-400 µL available volumes). Next, the samples were evaporated to dryness (using a Turbo Vap evaporator) to remove the organic solvent. Samples were then prepared depending on further analysis, either liquid chromatography coupled to high-resolution mass spectrometry (LC/MS) or gas chromatography coupled to high-resolution mass spectrometry (GC/MS). The samples were run in a randomized fashion on all platforms, GC and LCs.

### 2. Acquisition of the metabolic profiles

### 2.1. LC-MS/MS

Samples were reconstituted with 150 µL of deionized water/acetonitrile (95/5, v/v). The analysis were performed using a Prominence UFLC device from Shimadzu (human sera and mice plasmas) or Water Acquity (human plasmas). The samples were separated on a 150 x 2.1 mm Hypersil Gold Cs (1.9 µm) column (Thermo Fisher Scientific) and each analysis was carried out at a flow rate of 500 µL /min with mobile phases A (deionized water) and B (acetonitrile), both containing 0.1% formic acid. Gradient consisted of an isocratic step of 2 min at 95% phase A, followed by a linear gradient from 5 to 100% of phase B during the next 11 min, then followed by an isocratic step of 12.5 min. at 100% phase B, before returning to 95% phase A during 4.5 min. The mass spectrometer (Exactive, Orbitrap technology from Thermo Fisher Scientific) was fitted with a heated electrospray ionization source. The metabolite profiling ESI-MS experiments were successively performed in both positive and negative ion detection modes. The mass spectra were recorded using a mass resolution of 50 000 FWHM in the Exactive analyzer. Control and AD sample analysis were totally randomized during LC/MS experiments. Blank injections consisted of a mixture of H2O/ACN (95/5, v/v).

### 2.2. GC/MS.

The samples destined for GC/MS analysis were re-dried under vacuum desiccation for a minimum of 24 hours prior to being derivatized under dried nitrogen using bistrimethyl-silyl-triflouroacetamide (BSTFA). The GC column was 5% phenyl and the temperature ramp was from 40° to 300° C in a 16 minute period. Samples were analyzed on a Thermo-Finnigan Trace DSQ fast-scanning single-quadrupole mass spectrometer using electron impact ionization. The instrument was tuned and calibrated for mass resolution and mass accuracy on a daily basis.

### 3. LC/MS and/or GC/MS raw data treatment.

### 3.1. Human sera and mice plasmas

The LC/MS metabolome profiles were acquired using Xcalibur version 2.1 software. The data processing pipeline including filtering, feature detection and chromatographic peaks alignment was achieved by using the XCMS open-access software (Scripps Center, La Jola, CA, USA). A peak list was generated for further processing by statistical analysis.

### 3.2. Human plasmas

The hardware and software foundations for these informatics components were the LAN backbone, and a database server running Oracle 10.2.0.1 Enterprise Edition.

### 4. Identification of metabolites discriminating Alzheimer's disease versus control samples.

In order to identify metabolites that discriminate Alzheimer patients versus controls in the human serum first set of samples, each of the 3,537 variables that passed the quality-control treatment were analyzed with an Analysis-of-the-Variance (ANOVA) adjusted on the age, according to the following linear model:
*yᵢ∼Status* + *Age* + *Gender* with *yᵢ* corresponding to each of the 3,537 variables. A cutoff of 0.005 on the resulting p-values (corresponding to a local False-Discovery-Rate of 5% [27]) selected 795 significant variables.

Classification performance models from single or combination of variables were evaluated with AUC, sensitivity and specificity based on a Linear-Discriminant-Analysis (LDA) repeated random subsampling validation in order to avoid overfitting (carte R package [28]).

### 5. Biomarker identification; open-access databases request.

The annotations of the raw variables according to the mass m/z of each signal were performed by the request of open-access metabolite databases such as KEGG, HMDB, Metlin or Lipid Maps. These automatic annotations were performed on every variable considered as associated with a molecular peak ([M+H]+ or [M-H]- in positive or negative modes, respectively). Molecular peaks, secondary peaks or adducts associated with the same metabolite were thereby identified by automatic annotation.

The signals of interest, associated with discriminative and/or annotated variables, were validated in mass spectra of random samples (AD, MCI and control samples) by using the software Xcalibur 2.1 (Thermo Fischer Scientific).

Unidentified compounds have the potential to be identified by future acquisition of a matching purified standard or by classical structural analysis.

### 6. Characterization of metabolites of interest: analysis and fragmentation of targeted signals.

Some of the metabolites considered as putative biomarkers were re-analyzed in the same chromatographic conditions as used for acquisition of the metabolic profiles, except that the LC/MS-MS device is an UPLC Accela chromatography instrument coupled to a LTQ-Orbitrap Discovery mass spectrometer (Thermo Fisher Scientific).

CID (Collision Induced Dissociation) spectra (*i.e.* fragmentation spectra) were realized for each targeted signal if present in sufficient amount. The fragmentation was done on full scan of biological samples and a data-dependent (using a standard molecule) event at 7 500 FWHM. Three collision energies were used at 20, 30 and 40 (arbitrary unit). All full scan and CID spectra were interpreted using the software Xcalibur 2.1 (Thermo Fischer Scientific).

Handmade identification of putative metabolites from the results of fragmentation products was performed.

When a standard compound was available, an accurate identification of the molecule corresponding to the mass and to retention time was possible.

### 7. Metabolites identification

Thirty one sera metabolites, corresponding to 45 primary adducts for positive or negative mode, have been identified from this analysis (table 5). The observed m/z value, the approximate retention time and the monoisotopic mass given in tables 1 and 2 allow to easily identify the biomarkers within a sample of a patient. Observed m/z values can be subjected to variations up to 15 ppm due to the precision of the method used to analyze the sample. Approximate retention times are given to facilitate metabolites detection but several other methods can be used for the dosage of those metabolites.

**Table 5**

| **Metabolite** | **M/z ((M+H) or (M-H))** | **Retention time (min)** | **Ionisation mode** | **P-value** | **Variation in AD (in respect to control sample)** |
|---|---|---|---|---|---|
| **Azelaic acid** | 189,11139 | 6,09598 | + | 4,59E-09 | increase |
| **Azelaic acid** | 187,09608 | 6,07627 | - | 4,57E-07 | increase |
| **Dodecanedioic acid** | 229,14336 | 7,76616 | - | 3,88E-03 | increase |
| **Sebacic acid** | 203,12714 | 6,69376 | + | 1,38E-10 | increase |
| **Sebacic acid** | 201,11236 | 6,67086 | - | 1,06E-07 | increase |
| **Hippuric acid** | 180,06510 | 4,62134 | + | 3,21E-02 | decrease |
| **Hippuric acid** | 178,04986 | 4,59374 | - | 1,85E-02 | decrease |
| **Tryptophan** | 203,08168 | 3,89386 | - | 4,76E-04 | decrease |
| **Tryptophan** | 205,09669 | 3,94109 | + | 3,80E-05 | decrease |
| **Tyrosine** | 182,08092 | 1,22084 | + | 6,29E-05 | decrease |
| **Phenylacetylglutamine** | 265,11725 | 4,72844 | + | 1,35E-02 | increase |
| **Phenylacetylglutamine** | 263,10342 | 4,70433 | - | 1,10E-02 | increase |
| **Caproic acid** | 115,07516 | 4,69762 | - | 8,57E-05 | increase |
| **Iso-valeric acid** | 101,05930 | 3,44798 | - | 8,24E-05 | increase |
| **Caffeine** | 195,08706 | 4,49411 | + | 2,76E-03 | decrease |
| **L-Citrulline** | 176,10229 | 0,85602 | + | 3,00E-02 | decrease |
| **PFAM (22:1)** | 338,34022 | 13,69282 | + | 7,90E-28 | decrease |
| **4-Methyl-2-oxovaleric acid** | 129,05427 | 4,41515 | - | 2,93E-02 | decrease |
| **PFAM (20:1)** | 310,30909 | 13,01947 | + | 1,11E-35 | decrease |
| **PFAM (22:2)** | 336,32463 | 13,17333 | + | 1,15E-32 | decrease |
| **Theophylline and/or paraxanthine*** | 181,07141 | 3,67416 | + | 8,63E-04 | decrease |
| **Valeric acid** | 101,05930 | 4,41422 | - | 2,14E-02 | decrease |
| **Aminoisobutyric acid** | 102,05464 | 0,84354 | - | 3,36E-03 | increase |
| **Aminoisobutyric acid** | 104,07150 | 0,87952 | + | 2,74E-04 | decrease |
| **Aspartate** | 132,02883 | 0,85020 | - | 5,01E-08 | increase |
| **Asp-Phe** | 281,11195 | 4,08528 | + | 9,12E-13 | increase |
| **Asp-Phe** | 279,09810 | 4,05148 | - | 1,09E-12 | increase |
| **Undecanedioic acid** | 217,14292 | 7,25432 | + | 5,17E-09 | increase |
| **Undecanedioic acid** | 215,12814 | 7,23320 | - | 1,86E-07 | increase |
| **1-Monopalmitin** | 331,28362 | 12,13150 | + | 6,78E-07 | increase |
| **9,12-dioxo-dodecanoic acid** | 229,14282 | 7,16655 | + | 8,63E-05 | increase |
| **9,12-dioxo-dodecanoic acid** | 227,12787 | 7,14630 | - | 2,16E-03 | increase |
| **Nonenedioic acid** | 187,09584 | 5,19255 | + | 5,62E-07 | increase |
| **Nonenedioic acid** | 185,08082 | 5,17398 | - | 1,10E-05 | increase |
| **Octadecadienoyl-glycero-3- phosphate** | 433,23620 | 10,69926 | - | 3,71E-03 | increase |
| **Sulfobenzylalcohol** | 187,00591 | 5,31783 | - | 1,51E-03 | increase |
| **Glycocholic acid** | 466,31603 | 7,58454 | + | 1,29E-02 | decrease |
| **Glycocholic acid** | 464,29950 | 7,54681 | - | 5,78E-03 | decrease |
| **Guanosine** | 284,09824 | 1,15543 | + | 2,00E-10 | decrease |
| **Guanosine** | 282,08365 | 1,14429 | - | 1,40E-08 | decrease |
| **Inosine** | 269,08695 | 1,23465 | + | 9,99E-08 | decrease |
| **Inosine** | 267,07274 | 1,18228 | - | 1,10E-07 | decrease |
| **L-Threonic acid** | 135,02841 | 0,84726 | - | 6,96E-03 | decrease |
| **Ser-Phe** | 253,11788 | 1,79742 | + | 1,75E-07 | decrease |
| **Ser-Phe** | 251,10285 | 1,73240 | - | 6,54E-08 | decrease |

| | | | | | |
|---|---|---|---|---|---|
| *biomarker corresponds to theophylline or paraxanthine or a mix thereof; | | | | | |

A LC/MS analysis similar to that applied to human set 1 sera sample was also performed on blood sample from Tg2576 mouse, a transgenic model for AD, and as stated above, on a second human serum collection. Results have been replicated in the other human set of sera samples for 17 of the firstly identified metabolites. Interestingly, mass peaks corresponding to 7 of these 17 metabolites have been shown statistically associated to the disease status of the transgenic animals and moreover their quantity varies in the same way than in humans (table 6). The human plasma analysis (LC/MS - GC/MS) has confirmed usefulness of the biomarkers identified during the sera analysis.

**Table 6**

| **Metabolites replicated in human** | **Level variation (AD versus CTRL)** | **Level variation when replicated in Mouse** |
|---|---|---|
| **Azelaic acid** | increase | increase |
| **4-Methyl-2-oxovaleric acid** | decrease | decrease |
| **Dodecanedioic acid** | increase | increase |
| **Tryptophan** | decrease | decrease |
| **PFAM (22:1)** | decrease | decrease |
| **PFAM (20:1)** | decrease | decrease |
| **PFAM (22:2)** | decrease | decrease |
| **Sebacic acid** | increase | n/a |
| **Hippuric acid** | decrease | n/a |
| **Tyrosine** | decrease | n/a |
| **Phenylacetylglutamine** | increase | n/a |
| **Caproic acid** | increase | n/a |
| **Iso-valeric acid** | increase | n/a |
| **Caffeine** | decrease | n/a |
| **L-Citrulline** | decrease | n/a |
| **Theophylline and/or paraxanthine*** | decrease | n/a |
| **Valeric acid** | decrease | n/a |

| | | |
|---|---|---|
| *biomarker corresponds to theophylline or paraxanthine or a mix thereof; n/a: not available | | |

### B) Results analysis

### 1. Sets of biomarkers of the invention allow an accurate and efficient diagnostic of AD and related disorders.

Though biomarkers of the invention are particularly efficient for diagnosing AD and related disorders when used alone, the use of sets of at least two biomarkers is of interest in order to increase the sensitivity of diagnostic tests.

In order to set up efficient sets of biomarkers (classifiers) for the diagnostic of AD and related disorders, a linear discriminant analysis was performed [29].

AUC, sensitivity and specificity were computed as the mean of 100 resampling iterations. For each iteration, 2/3 of the samples were used to train the classifier, and the remaining 1/3 were used to test the classifier and to provide AUC, sensitivity and specificity estimates.

Inventors have been able to identify several sets of biomarkers of the invention with satisfying sensitivity and specificity which are listed in table 7. Sensitivity is the proportion of subjects who are correctly categorized as having disease among those who truly have the disease. Similarly, specificity is the proportion of subjects who are correctly categorized as not having the disease among all subjects who truly don't have the disease. Noteworthy, sensitivity above 80% is observed for more than 50% of these sets.

**Table 7**

| **Set of biomarkers** | **AUC** | **Sensitivity** | **Specificity** |
|---|---|---|---|
| Asp-Phe + Ser-Phe | **93** | **88** | **84** |
| Tryptophan + Asp-Phe + Caproic acid | **88** | **85** | **80** |
| Azelaic acid + Asp-Phe + L-Threonic acid | **87** | **81** | **80** |
| L-Citrulline + Iso-valeric acid + Aspartate | **85** | **81** | **81** |
| Caproic acid + Ser-Phe + Undecanedioic acid | **89** | **83** | **83** |
| L-Citrulline + Inosine + Aspartate + Ser-Phe | **91** | **90** | **80** |
| Tyrosine + 1-Monopalmitin + Aspartate + Ser-Phe | **90** | **88** | **80** |
| Nonenedioic acid + Tryptophan + Asp-Phe + L-Threonic acid | **88** | **80** | **81** |
| Theophylline and/or Paraxanthine* + Asp-Phe + L-Threonic acid + Sebacic acid | **87** | **80** | **81** |
| L-Citrulline + Tryptophan + Aspartate + L-Threonic acid | **86** | **81** | **82** |
| Nonenedioic acid + Undecanedioic acid + Sulfobenzylalcohol + Ser-Phe | **86** | **80** | **80** |
| L-Citrulline + Undecanedioic acid + Aspartate + Sulfobenzylalcohol | **85** | **81** | **80** |
| L-Citrulline + Azelaic acid + Aspartate + Glycocholic acid | **84** | **81** | **80** |
| L-Citrulline + Theophylline and/or paraxanthine* + Azelaic acid | **83** | **83** | **75** |
| L-Citrulline + Azelaic acid + Valeric acid + Phenylacetylglutamine | **83** | **80** | **75** |
| L-Citrulline + Hippuric acid + Sebacic acid + Dodecanedioic acid + Tryptophan | **83** | **80** | **77** |
| L-Citrulline + Azelaic acid + Tryptophan + 4-Methyl-2-oxovaleric acid | **83** | **80** | **75** |
| L-Citrulline + Azelaic acid + Tryptophan + Iso-valeric acid + Phenylacetylglutamine | **83** | **82** | **75** |
| L-Citrulline + Tyrosine + Azelaic acid + Tryptophan + Iso-valeric acid | **83** | **80** | **75** |
| PFAM (20:1) + PFAM (22:2) | **98** | **89** | **92** |
| PFAM (20:1) + PFAM (22:1) | **99** | **93** | **94** |
| PFAM (22:2) + PFAM (22:1) | **97** | **94** | **89** |
| PFAM (20:1) + PFAM (22:2) + PFAM (22:1) | **99** | **94** | **94** |
| Caffeine + Asp-Phe + Ser-Phe | **92** | **88** | **81** |
| Asp-Phe + Dodecanedioic acid + Ser-Phe | **93** | **89** | **82** |
| Asp-Phe + Guanosine + Ser-Phe | **93** | **89** | **82** |
| Asp-Phe + Hippuric acid + Ser-Phe | **92** | **86** | **85** |
| Asp-Phe + 4-Methyl-2-oxovaleric acid + Ser-Phe | **92** | **89** | **82** |
| Asp-Phe + Ser-Phe + Octadecadienoyl-glycero-3-phosphate | **92** | **88** | **81** |
| 9,12-dioxo-dodecanoic acid + Asp-Phe + Ser-Phe | **92** | **88** | **82** |
| Asp-Phe + Ser-Phe + Phenylacetylglutamine | **92** | **87** | **80** |
| Azelaic acid + Dodecanedioic acid | **78** | **78** | **67** |
| Azelaic acid + Sebacic acid | **78** | **78** | **67** |
| Azelaic acid + Sebacic acid + Dodecanedioic acid | **79** | **79** | **68** |
| Azelaic acid + Undecanedioic acid | **80** | **78** | **70** |
| Azelaic acid + Undecanedioic acid + Dodecanedioic acid | **78** | **78** | **67** |
| Azelaic acid + Undecanedioic acid + Sebacic acid | **80** | **77** | **72** |
| Azelaic acid + Undecanedioic acid + Sebacic acid + Dodecanedioic acid | **76** | **78** | **66** |
| Nonenedioic acid + Azelaic acid | **79** | **78** | **68** |
| Nonenedioic acid + Azelaic acid + Dodecanedioic acid | **79** | **76** | **69** |
| Nonenedioic acid + Azelaic acid + Sebacic acid | **79** | **74** | **69** |
| Nonenedioic acid + Azelaic acid + Sebacic acid + Dodecanedioic acid | **77** | **75** | **67** |
| Nonenedioic acid + Azelaic acid + Undecanedioic acid | **80** | **76** | **69** |
| Nonenedioic acid + Azelaic acid + Undecanedioic acid + Dodecanedioic acid | **79** | **74** | **68** |
| Nonenedioic acid + Azelaic acid + Undecanedioic acid + Sebacic acid | **78** | **75** | **68** |
| Nonenedioic acid + Azelaic acid + Undecanedioic acid + Sebacic acid + Dodecanedioic acid | **76** | **74** | **64** |
| Nonenedioic acid + Dodecanedioic acid | **75** | **73** | **68** |
| Nonenedioic acid + Sebacic acid | **77** | **72** | **72** |
| Nonenedioic acid + Sebacic acid + Dodecanedioic acid | **75** | **70** | **68** |
| Nonenedioic acid + Undecanedioic acid | **79** | **74** | **68** |
| Nonenedioic acid + Undecanedioic acid + Dodecanedioic acid | **78** | **74** | **67** |
| Nonenedioic acid + Undecanedioic acid + Sebacic acid | **79** | **75** | **69** |
| Nonenedioic acid + Undecanedioic acid + Sebacic acid + Dodecanedioic acid | **76** | **72** | **67** |
| Sebacic acid + Dodecanedioic acid | **76** | **72** | **69** |
| Undecanedioic acid + Dodecanedioic acid | **79** | **78** | **71** |
| Undecanedioic acid + Sebacic acid | **78** | **77** | **70** |
| Undecanedioic acid + Sebacic acid + Dodecanedioic acid | **77** | **75** | **68** |
| Sebacic acid + Tyrosine | **84** | **78** | **75** |
| Hippuric acid + Sebacic acid + Tyrosine | **84** | **79** | **76** |
| Sebacic acid + Tyrosine + Undecanedioic acid | **83** | **77** | **74** |
| Sebacic acid + Tryptophan + Tyrosine | **85** | **80** | **75** |
| Sebacic acid + Tryptophan | **85** | **85** | **74** |
| Hippuric acid + Sebacic acid + Tyrosine + Undecanedioic acid | **83** | **79** | **75** |
| Dodecanedioic acid + Sebacic acid + Tyrosine + Undecanedioic acid | **82** | **75** | **72** |
| Dodecanedioic acid + Sebacic acid + Tyrosine | **82** | **75** | **73** |
| Sebacic acid + Tryptophan + Undecanedioic acid | **84** | **85** | **73** |
| Dodecanedioic acid + Sebacic acid + Tryptophan + Tyrosine | **83** | **78** | **74** |
| Hippuric acid + Sebacic acid + Tryptophan + Tyrosine | **85** | **85** | **77** |
| Dodecanedioic acid + Sebacic acid + Tryptophan | **84** | **85** | **74** |
| Dodecanedioic acid + Hippuric acid + Sebacic acid + Tyrosine + Undecanedioic acid | **82** | **78** | **74** |
| Hippuric acid + Sebacic acid | **84** | **82** | **71** |
| Dodecanedioic acid + Hippuric acid + Sebacic acid + Tyrosine | **83** | **79** | **73** |
| Sebacic acid + Tryptophan + Tyrosine + Undecanedioic acid | **83** | **81** | **73** |
| Hippuric acid + Sebacic acid + Tryptophan + Undecanedioic acid | **85** | **86** | **75** |
| Hippuric acid + Sebacic acid + Tryptophan | **85** | **87** | **76** |
| Dodecanedioic acid + Sebacic acid + Tryptophan + Undecanedioic acid | **83** | **84** | **73** |
| Hippuric acid + Sebacic acid + Tryptophan + Tyrosine + Undecanedioic acid | **84** | **83** | **75** |
| Dodecanedioic acid + Sebacic acid + Tryptophan + Tyrosine + Undecanedioic acid | **83** | **80** | **73** |
| Dodecanedioic acid + Hippuric acid + Sebacic acid + Tryptophan + Tyrosine | **84** | **84** | **76** |
| Dodecanedioic acid + Hippuric acid + Sebacic acid | **82** | **81** | **68** |
| Hippuric acid + Sebacic acid + Undecanedioic acid | **83** | **82** | **70** |
| Dodecanedioic acid + Hippuric acid + Sebacic acid + Tryptophan | **84** | **86** | **75** |
| Dodecanedioic acid + Hippuric acid + Sebacic acid + Undecanedioic acid | **81** | **81** | **67** |
| Dodecanedioic acid + Hippuric acid + Sebacic acid + Tryptophan + Tyrosine + Undecanedioic acid | **82** | **82** | **72** |
| Dodecanedioic acid + Sebacic acid | **81** | **81** | **69** |
| Dodecanedioic acid + Hippuric acid + Tryptophan | **76** | **78** | **68** |
| Dodecanedioic acid + Hippuric acid | **69** | **71** | **63** |
| Dodecanedioic acid + Hippuric acid + Sebacic acid + Tryptophan + Undecanedioic acid | **83** | **84** | **74** |
| Dodecanedioic acid + Tyrosine | **73** | **68** | **67** |
| Dodecanedioic acid + Tryptophan | **74** | **74** | **61** |
| Dodecanedioic acid + Hippuric acid + Tyrosine | **72** | **70** | **68** |
| Dodecanedioic acid + Hippuric acid + Tryptophan + Undecanedioic acid | **82** | **80** | **73** |
| Hippuric acid + Tryptophan | **75** | **74** | **64** |
| Dodecanedioic acid + Hippuric acid + Undecanedioic acid | **80** | **78** | **68** |
| Dodecanedioic acid + Hippuric acid + Tryptophan + Tyrosine | **76** | **75** | **71** |
| Dodecanedioic acid + Hippuric acid + Tyrosine + Undecanedioic acid | **80** | **76** | **71** |
| Dodecanedioic acid + Tryptophan + Tyrosine | **75** | **73** | **69** |
| Dodecanedioic acid + Tryptophan + Undecanedioic acid | **81** | **83** | **69** |
| Dodecanedioic acid + Tyrosine + Undecanedioic acid | **80** | **75** | **68** |
| Hippuric acid + Tyrosine + Undecanedioic acid | **82** | **76** | **71** |
| Hippuric acid + Tryptophan + Tyrosine | **75** | **68** | **71** |
| Hippuric acid + Tryptophan + Undecanedioic acid | **84** | **81** | **73** |
| Hippuric acid + Undecanedioic acid | **81** | **78** | **68** |
| Hippuric acid + Tryptophan + Tyrosine + Undecanedioic acid | **83** | **79** | **72** |
| Hippuric acid + Tyrosine | **72** | **61** | **69** |
| Dodecanedioic acid + Tryptophan + Tyrosine + Undecanedioic acid | **81** | **80** | **68** |
| Dodecanedioic acid + Hippuric acid + Tryptophan + Tyrosine + Undecanedioic acid | **81** | **78** | **70** |
| Tyrosine + Undecanedioic acid | **81** | **75** | **69** |
| Tryptophan + Undecanedioic acid | **83** | **85** | **70** |
| Tryptophan + Tyrosine + Undecanedioic acid | **83** | **80** | **69** |
| Tryptophan + Tyrosine | **73** | **63** | **65** |
| Asp-Phe + Valeric acid + Ser-Phe | **92** | **87** | **83** |

| | | | |
|---|---|---|---|
| *biomarker corresponds to theophylline or paraxanthine or a mix thereof; AUC: Area Under the Curve. | | | |

### 2. Biomarkers of the invention allow subclassification of patients.

Table 8 gives, for the most significant biomarkers selected as explained above, an estimated deviation in patient suffering from MCI or AD expressed as a percentage of the level measured in control (first set of human sera samples).

Interestingly when a selected biomarker was found decreased in AD, it was also found decreased in MCI patients (table 8), and conversely an increased biomarker in AD was found increased in MCI patients.

Hence biomarkers of the invention provide tools for diagnosing AD and related disorders but also for predicting the risk for a patient of conversion from MCI to established AD.

**Table 8**

| **Metabolite** | **P-value** | **Variation in AD (% of control)** | **AUC (AD)** | **Sensibility (AD)** | **Sensitivity (AD)** | **Variation in MCI (% of control)** |
|---|---|---|---|---|---|---|
| 1-monopalmitin | 6,78E-07 | increase *** | 76,3 | 68,8 | 65 | increase |
| 9,12-dioxo-dodecanoic acid | 8,63E-05 | increase *** | 69,9 | 61,7 | 66,8 | increase * |
| Aminoisobutyric acid | 2,74E-04 | decrease *** | 67,9 | 60 | 67,9 | decrease |
| Aspartate | 5,01E-08 | increase *** | 80 | 74,4 | 67 | increase ** |
| Asp-Phe | 9,12E-13 | increase *** | 84,8 | 76,2 | 76,7 | increase * |
| Azelaic acid | 4,59E-09 | increase *** | 79,9 | 79,9 | 67,5 | increase * |
| Caffeine | 2,76E-03 | decrease ** | 64,9 | 68,5 | 52,9 | decrease |
| Caproic acid | 8,57E-05 | increase *** | 66,8 | 62,5 | 55,2 | increase |
| Dodecanedioic acid | 3,88E-03 | increase ** | 68,8 | 67,8 | 68,4 | increase *** |
| Glycocholic acid | 5,78E-03 | decrease * | 65,5 | 59,4 | 66,1 | decrease * |
| Guanosine | 2,00E-10 | decrease *** | 80,3 | 84,8 | 66,4 | decrease |
| Hippuric acid | 1,85E-02 | decrease * | 63 | 53,8 | 67 | decrease |
| Inosine | 9,99E-08 | decrease *** | 76,6 | 77,2 | 67,1 | decrease |
| Iso-valeric acid | 8,24E-05 | increase *** | 68,7 | 61,2 | 61 | increase * |
| L-citrulline | 3,00E-02 | decrease * | 60,7 | 52,1 | 63,2 | decrease |
| L-threonic acid | 6,96E-03 | decrease * | 62,6 | 57,1 | 59,9 | decrease |
| Nonenedioic acid | 5,62E-07 | increase *** | 75,5 | 71,8 | 70,4 | increase * |
| Octadecadienoyl-glycero-3-phosphate | 3,71E-03 | increase ** | 66,6 | 59,3 | 64,5 | increase |
| PFAM (20:1) | 1,11E-35 | decrease *** | 97,6 | 90,3 | 91,2 | decrease *** |
| PFAM (22:1) | 7,90E-28 | decrease *** | 95,5 | 91,4 | 85,7 | decrease *** |
| PFAM (22:2) | 1,15E-32 | decrease *** | 96,6 | 90,8 | 89,5 | decrease *** |
| Sebacic acid | 1,38E-10 | increase *** | 82,7 | 80,9 | 71,4 | increase * |
| Ser-Phe | 6,54E-08 | decrease *** | 77,9 | 78,8 | 63,7 | decrease * |
| Sulfobenzylalcohol | 1,51E-03 | increase ** | 65,1 | 53,4 | 70,1 | increase |
| Tryptophan | 3,80E-05 | decrease *** | 72,1 | 65,4 | 58,2 | decrease |
| Tyrosine | 6,29E-05 | decrease *** | 70,1 | 59,5 | 65,4 | decrease * |
| Undecanedioic acid | 5,17E-09 | increase *** | 79,8 | 76,8 | 72,1 | increase * |

| | | | | | | |
|---|---|---|---|---|---|---|
| ***: differences between control and AD patients or MCI patients and control are significant with a p-value < 0.001; **: p-value < 0.005; *: p-value < 0.05. | | | | | | |

### 3. Biomarkers of the disclosure can vary as a function of the response to treatment.

Drugs targeting the cholinergic system (for instance acetylcholinesterase inhibitors: donepezil, rivastigmine or galantamine) or NMDA inhibitors (as memantine) are the sole medications currently approved and given to AD patients to counter neurological symptoms of AD. Table 9 below lists biomarkers which have been found to vary significantly as a function of the presence of such treatments. Observed levels for these biomarkers are at an intermediate position when compared to the diseased and the healthy subjects (fig. 1 and 2). This is also observed in the serum of MCI patients (not shown). Biomarkers used in the invention are thus efficient in monitoring the response of patients to the treatments. Interestingly, the deviation of some of these biomarkers is further specific to a given treatment.

**Table 9**

| **Metabolite** | **Association with treatment** | |
|---|---|---|
| | **AchE Inhibitors** | **Memantine** |
| **Asp-Phe** | + | + |
| **Glycocholic acid** | - | + |
| **Guanosine** | + | + |
| **Inosine** | + | + |
| **Tyrosine** | - | + |
| **PFAM (20:1)** | + | - |
| **PFAM (22:1**) | + | - |
| **PFAM (22:2)** | + | - |

| | | |
|---|---|---|
| +: Expression level of the biomarker is significantly correlated with treatment; -: Expression level of the biomarker is not significantly correlated with treatment; n/a: data not available. | | |

### C. Quantification of biomarkers

A quantitative analysis is conducted on the first set of human serum samples (see A) 1.Human serum samples). Three biomarkers of interest, namely sebacic acid, dodecanedioic acid and tryptophan, are significantly differentially quantified in AD sera as compared to control samples.

### 1. MATERIAL AND METHODS

### 1.1. Biological samples

The study was conducted on the two groups of human serum first set of samples.

### 1.2. Tissue sampling: serum collection

A volume of 8 mL serum separator tubes (SSTs) was required to collect human blood samples. The selection criteria of the samples were the same than previously described.

### 1.3. Standards and reagents

The following pure non-labelled and labelled (internal) standards of the three metabolites of interest were purchased to Sigma-Aldrich:
- L-tryptophan, formula C₁₁H₁₂N₂O₂
- L-tryptophan-D5(indole-d5), formula C₁₁D₅H₇N₂O₂
- dodecanedioic acid, formula C₁₂H₂₂O₄
- dodecanedioic acid-1,12-¹³C₂, formula ¹³C₂C₁₀H₂₂O₄,
- sebacic acid, formula C₁₀H₁₈O₄
- sebacic acid-d16, formula C₁₀D₁₆H₂O₄.

The organic solvents for HPLC gradient grade were: methanol (MeOH) [VWR Prolabo, HiPer-Solv CHROMANORM, ref. 20864.320] and acetonitrile (ACN) [Sigma-Aldrich, Chromasolv, ref. 34851-2.5L]. The formic acid (HCOOH) added in solvent was of 99-100% purity (VWR Prolabo, AnalaR NORMAPUR). The water was in-house ultra-pure water (H₂O) (USF Elga, Maxima II).

### 1.4. Instrumentation

The metabolic signals were acquired in negative ionization mode using a Q-Exactive mass spectrometer (Orbitrap technology) fitted with a new heated electrospray ion source HESI-II (Thermo Fisher Scientific, San Jose, CA, USA). Liquid chromatographic separations were performed using an ultra-high performance liquid chromatography (UHPLC) Transcend device (Thermo Fisher Scientific, San Jose, CA, USA). The system was operated on Xcalibur software (version 2.2, Thermo Fischer Scientific).

### 1.4.1. Ion source (tune) parameters

The parameters were the following: sheath gas = 80 (AU), auxiliary gas = 20 (AU), sweep gas = 0 (AU), spray voltage = |2.50| kV, capillary temperature = 380°C, S-lens radio-frequence = 70 (AU), heater temperature = 200°C. Table 10 summarizes intensities of each compound for these optimized parameters.

**Table 10: Ion source parameters**

| **Standard** | **Mass of theorical signal [M-H]⁻ (m/z)** | **Intensity of signal (average over 0.30 min)** |
|---|---|---|
| Tryptophan | 203.0826009 | 1.32E+08 |
| Sebacic acid | 201.1132323 | 2.05E+09 |
| Dodecanedioic acid | 229.1445324 | 1.39E09 (*Nb*: Cap. T°C = 400°C & Heater = 360°C) |

### 1.4.2. Fragmentation (HCD) parameters

Table 11 summarizes the Higher-energy Collisional-induced Dissociation (HCD) parameters (i.e. Normal Collision Energy (NCE) values) obtained for each standard and the transition used for the specific quantification of each compound.

**Table 11: Fragmentation (HCD) parameters**

| **Standard** | **Precursor signal (m/z)** | **Scan range (m/z)** | **Daughter signal for quantification (m/z)** | **Neutral loss** | **NCE (arbitrary unit)** |
|---|---|---|---|---|---|
| Tryptophan | 203.0826009 | 50-225 | 116.05072 | C₃H₅NO₂ | 33 |
| Sebacic acid | 201.1132323 | 50-225 | 183.10275 | H₂O | 32 |
| Dodecanedioic acid | 229.1445324 | 50-250 | 211.13425 | H₂O | 45 |
| Tryptophan-d5 | 208.1139847 | 50-230 | 121.0821 | C₃H₅NO₂ | 42 |
| Sebacic acid-d16 | 217.2136603 | 50-240 | 153.20091 | CD₂O₃ | 37 |
| Dodecanedioic acid-2x13C | 231.151242 | 50-255 | 168.14766 | [13]CH₂O₃ | 64 |

### 1.4.3. Liquid chromatography (LC) method

A specific chromatographic method was developed for this study. The Kinetex C8 chromato-graphic column was chosen and set at 60°C. For each sample, 10 µL was injected into the instrument and the flow rate was set at 400 µL/min.

Mobile phases were composed of (A) ultra-pure water (H2O) and (B) high performance liquid chromatography (HPLC) grade acetonitrile (ACN) both containing 0.1 % formic acid (HCOOH). The gradient used is summarized in Table 12.

**Table 12: Chromatographic gradient conditions used for LC-MS experiments**

| **Time (min)** | **Duration (s)** | **% A** | **% B** | **Gradient type** |
|---|---|---|---|---|
| 0 to 2 | 120 | 95 | 5 | Isocratic |
| 2 to 8 | 360 | 37 | 63 | Ramp |
| 8 to 8.17 | 10 | 5 | 95 | Ramp |
| 8.17 to 13.17 | 300 | 5 | 95 | Isocratic |
| 13.17 to 13.18 | 1 | 95 | 5 | Ramp |
| 13.18 to 17 | 229 | 95 | 5 | Isocratic |

### 1.5. Preparation of the pools of standards

Two different pools were constituted ahead of the sample preparation. The first pool was a mix of the non-labelled standards and named pool_std. The second pool was a mix of the labelled (internal) standards and named pool_IS. Both pools were prepared as described below.

### 1.5.1. Pool of standards (pool_std)

The (non-labelled) standards were weighted and solubilized with an appropriate solvent. They were mixed together at a final concentration of 600X and stored at -20°C.

The final concentration in pool 600X was of 1800 µg/mL for tryptophan, 6 µg/mL for sebacic acid and 2.4 µg/mL for dodecanedioic acid.

From this pool_std 600X, thirteen daughter solutions were prepared by serial dilution in MeOH. These solutions were the following: 300X, 225X, 180X, 150X, 105X, 60X, 50X, 45X, 30X, 27X, 22.5X, 18X and 15X. The solutions 300X, 225X, 150X, 105X, 60X, 45X, 30X, 22.5X and 15X were used for the preparation of calibration sets (see paragraph B.1.6.2), whereas the solutions 180X, 50X, 27X and 18X were used for the preparation of their associated quality control (QC) samples.

### 1.5.2. Pool of internal standards (pool_IS)

The internal (labelled) standards (IS) were weighted and solubilized with an appropriate solvent. They were pooled together at a final concentration of 30X and stored at -20°C. The final concentration in pool 30X was of 180 µg/mL for tryptophan-d5, 0.9 µg/mL for sebacic acid-d16 and 1.2 µg/mL for dodecanedioic acid-2x13C.

This pool was used to spike with 5 µL all the samples before extraction (see paragraph B.1.6).

### 1.6. Schedule and ample preparation

The samples preparation was performed over 3 days. The schedule of the preparation of the sample is described in Table 13.

**Table 13: Schedule of sample preparation**

| | Day 1 | Day 2 | Day 3 |
|---|---|---|---|
| Biological samples | Preparation of first batch (54 samples) | Preparation of second batch (54 samples) | |
| Calibration curves | Preparation of CTRL1 calibration set and QC samples (1) | Preparation of CTRL1 calibration set and QC samples (2) | Preparation of CTRL2 calibration set and QC samples |

### 1.6.1. Biological samples

For each serum sample aliquot, an aliquot of 200 µL of frozen biofluid was thawed on the bench at room temperature (RT°) during 1 h [step 1] and vortexed 5 s [step 2]. For each sample, 50 µL was removed [step 3] and prepared as follow before LC-MS/MS acquisition:
- 50 µL of serum sample + 200 µL MeOH (= protein precipitation step) [step 4];
- Addition of 5 µL of the pool_IS 30x [step 5];
- 5 s vortex treatment [step 6];
- 5 min ultrasonic treatment at RT° [step 7];
- 5 s vortex treatment [step 8];
- 20 min under agitation (400 rpm) at RT° [step 9];
- 5 s vortex treatment [step 10];
- Centrifugation at 10 000 g at 4°C during 10 min [step 11];
- Recovery of the supernatant at RT° [step 12];
- Evaporation to dryness (N2 stream) at 30°C during 90 min [step 13];
- Dissolution in 150 µL of a mixture H2O/ACN/HCOOH (95/5/0.01, v/v/v) set at 4°C [step 14];
- 5 s vortex treatment [step 15];
- Centrifugation at 10 000 g at 4°C during 5 min [step 16];
- Recovery of 100 µL of the supernatant at RT° and transfer into a vial of injection [step 17].

Five "blanks" of extraction were prepared as described above with 50 µL of ultra-pure water placed into five collection tubes tubes at -80°C the day.

Of note, the final concentration of IS was 1X (added in each vial).

### 1.6.2. Calibration curves

Three calibration sets and their associated QC were prepared, two from a pool of serum sample and one in water.

### CTRL1 calibration set and QC samples

The main calibration set (n = 4) and its associated QC samples (n = 5) were realized from a pool of CTRL samples of the study conducted on set of human sera.

This pool was constituted during the day 1 of biological sample preparation (see paragraph 1.6.1). For this, 70 µL of each CTRL sample (n = 50) were removed from one aliquot and pooled together into a 5 mL collection tube.

During day 1, a volume of 50 µL of this pool was aliquoted in twenty-eight collection tubes to constitute a duplicate of calibration set and a duplicate of associated QC samples. The pool was then stored at -20°C after use.

During the day 2, the rest of the pool was thawed on the bench during 1 h. Then, a volume of 50 µL of this pool was aliquoted in thirty-two collection tubes to constitute another duplicate of calibration set and a triplicate of associated QC samples. Three replicates of the QC samples were prepared, two series being injected with their associated calibration curve and one serie injected between day 1 and day 2.

Four series (n = 4) of calibration set and its associated QC samples (four levels, n = 5) were realized.

The sixty aliquots were extracted as the biological samples (see paragraph 1.6.1), except that 5 µL of daughter solutions of pool_std 600X (see paragraph 1.5.1) were spiked during the step 5 of preparation.

As explained in the paragraph 1.6.1, a dilution of a factor of 30 was observed for each spiked standard into the final vial of each sample.

### CTRL2 calibration set and QC samples

A second calibration set (n = 1) and its associated QC samples (n = 4) were realized from a pool of CTRL samples of the human serum set.

This pool was constituted during the day 3 of biological sample preparation (i.e. the day after the preparation of the second batch, the 7th of November, 2013). For this, 50 µL of sixteen CTRL samples of the study conducted on the set of human sera were removed from an aliquot of 200 µL and pooled together into a 1.5 mL microtube.

The fourteen aliquots were extracted as the biological samples (see paragraph 1.6.1), except that 5 µL of the relevant daughter solutions of the solution "pool_std 600X" (see paragraph 1.5.1) were spiked during the step 5 of preparation.

### H20 calibration set

A calibration set (n = 1) was realized in water (H₂O) in order to quantify the targeted metabolites in the five blanks of extraction with an appropriate calibration set.

For this, 50 µL of (ultra-pure) water were placed into fourteen different collection tubes. These samples were extracted as the biological samples (see paragraph 1.6.1), but 5 µL of daughter solutions of pool_std 600X (see paragraph 1.5.1) were spiked during the step 6 of preparation. Indeed, to prepare ten levels of the calibration set, the aliquots noted: 10X, 7.5X, 5X, 3.5X, 2X, 1.5X, X, 0.75X and 0.5X were spiked respectively with the daughter solutions 300X, 225X, 150X, 105X, 60X, 45X, 30X, 22.5X and 15X.

### 1.7. Data bioprocessing

The signals were acquired with the Xcalibur software (version 2.2, Thermo Fischer Scientific, San Jose, CA, USA). The peaks were automatically integrated using a processing setup with the Xcalibur software 2.2 (Thermo Fischer Scientific). All the signals were smoothed by the Genesis algorithm and detected by "mass range" with a mass tolerance of 8 ppm.

All the signals in the different samples were detected and integrated by running the processing setup with the QuanBrowser part of the Xcalibur software 2.2.

### 1.8. Data treatment

The bioprocessed data were treated with Excel 2010 (Microsoft Office). Calibration curves, graphics and Student's t-test (AD versus CTRL samples, bilateral distributions and equal variances) were also realized with Excel 2010.

QC samples and Standard samples (i.e. samples used for the calibration sets) were validated if their respective percentage of difference (% Diff.) was below 15%. If a standard sample was not validated, it was deleted in order to plot the corrected calibration curves (i.e. after the correction of the respective endogenous concentration) with the best possible accuracy.

### 2. ANALYSIS OF TARGETED METABOLITES IN SERUM EXTRACTS

### 2.1. Sample preparation, acquisition and bioprocessing of data

The samples were prepared as detailed in the paragraph 1.6. To summarize, the biological samples were divided in two batches of 54 samples prepared over two days. A duplicate of the calibration curve (with its associated QC samples) from the CTRL1 pool was realized for each batch of samples. Furthermore, a calibration curve (with its associated QC samples) prepared from a pool of CTRL2 samples (as explained in the paragraph 1.6.2) and a calibration curve prepared from ultra-pure water were prepared, respectively during day 3 and 2.

The samples were randomized during the preparation and the acquisition.

### 2.2. Results

Results are presented in figure 3. The concentrations of the three biomarkers of interest are significantly different from those of the control. Sebacic acid was found in AD patient's sera at a mean concentration 87.6 ± 5.1 ng/mL of whereas is was of 58.4 ± 2.4 ng/mL in controls (+50% the control value). Dodecanedioic acid was also significantly increased in AD patient's sera with a mean concentration of 13.1 ± 1.4 ng/mL whereas it was of 8.2 ± 0.7 ng/mL in controls (+60% the control value). Tryptophan was decreased in AD patient's sera with a mean concentration of 2832 ± 108 ng/mL and a mean concentration of 3606 ± 139 ng/mL in controls (-21% the control value).

These results confirm those obtained in the above presented semi-quantitative studies (cf. section B)) and can serve as values helpful to diagnose AD in a subject suspected to suffer or to be at risk of AD.

Hence, biomarkers and sets thereof are suitable to diagnose, to survey the evolution, to evaluate the severity of AD or a related disorder and to assess the efficacy of the treatment thereof.

Biomarkers of the invention can be used for the development of companion tests for medication currently used or to be developed for AD.

### BIBLIOGRAPHY

1 Citron M (2004) Strategies for disease modification in Alzheimer's disease. Nat. Rev. Neurosci. 5, 677-85.
2 Suh Y-H & Checler F (2002) Amyloid precursor protein, presenilins, and alpha-synuclein: molecular pathogenesis and pharmacological applications in Alzheimer's disease. Pharmacol. Rev. 54, 469-525.
3 McKhann G, Drachman D, Folstein M, Katzman R, Price D & Stadlan EM (1984) Clinical diagnosis of Alzheimer's disease: report of the NINCDS-ADRDA Work Group under the auspices of Department of Health and Human Services Task Force on Alzheimer's Disease. Neurology 34, 939-44.
4 American Psychiatric Association (1997) Diagnostic and Statistical Manual of Mental Disorders, 4th ed. (American Psychiatric Association, ed.) U.S. National Library of Medicine, Washington, DC.
5 Petersen RC, Smith GE, Waring SC, Ivnik RJ, Tangalos EG & Kokmen E (1999) Mild cognitive impairment: clinical characterization and outcome. Arch. Neurol. 56, 303-8.
6 Albert MS, DeKosky ST, Dickson D, Dubois B, Feldman HH, Fox NC, Gamst A, Holtzman DM, Jagust WJ, Petersen RC, Snyder PJ, Carrillo MC, Thies B & Phelps CH (2011) The diagnosis of mild cognitive impairment due to Alzheimer's disease: recommendations from the National Institute on Aging-Alzheimer's Association workgroups on diagnostic guidelines for Alzheimer's disease. Alzheimers. Dement. 7, 270-9.
7 Thambisetty M & Lovestone S (2010) Blood-based biomarkers of Alzheimer's disease: challenging but feasible. Biomark. Med. 4, 65-79.
8 Gustaw-Rothenberg K, Lerner A, Bonda DJ, Lee H, Zhu X, Perry G & Smith MA (2010) Biomarkers in Alzheimer's disease: past, present and future. Biomark. Med. 4, 15-26.
9 Rupsingh R, Borrie M, Smith M, Wells JL & Bartha R (2011) Reduced hippocampal glutamate in Alzheimer disease. Neurobiol. Aging 32, 802-10.
10 Skoumalová A, Ivica J, Santorová P, Topinková E & Wilhelm J (2011) The lipid peroxidation products as possible markers of Alzheimer's disease in blood. Exp. Gerontol. 46, 38-42.
11 Gabelle A, Richard F, Gutierrez L-A, Schraen S, Delva F, Rouaud O, Buée L, Dartigues J-F, Touchon J, Lambert J-C & Berr C (2013) Plasma amyloid-β levels and prognosis in incident dementia cases of the 3-City Study. J. Alzheimers. Dis. 33, 381-91.
12 Pesini P, Pérez-Grijalba V, Monleón I, Boada M, Tárraga L, Martinez-Lage P, San-José I & Sarasa M (2012) Reliable Measurements of the β-Amyloid Pool in Blood Could Help in the Early Diagnosis of AD. Int. J. Alzheimers. Dis. 2012, 604141.
13 Toledo JB, Shaw LM & Trojanowski JQ (2013) Plasma amyloid beta measurements - a desired but elusive Alzheimer's disease biomarker. Alzheimers. Res. Ther. 5, 8.
14 Doecke JD, Laws SM, Faux NG, Wilson W, Burnham SC, Lam C-P, Mondal A, Bedo J, Bush AI, Brown B, De Ruyck K, Ellis KA, Fowler C, Gupta VB, Head R, Macaulay SL, Pertile K, Rowe CC, Rembach A, Rodrigues M, Rumble R, Szoeke C, Taddei K, Taddei T, Trounson B, Ames D, Masters CL & Martins RN (2012) Blood-based protein biomarkers for diagnosis of Alzheimer disease. Arch. Neurol. 69, 1318-25.
15 Aftab MF & Waraich RS (2012) A REVIEW OF BIOCHEMICAL MARKERS FOR EARLY DIAGNOSIS OF ALZHEIMERâ ™S DISEASE. Am. J. Neurosci. 3, 54-62.
16 Rosén C, Hansson O, Blennow K & Zetterberg H (2013) Fluid biomarkers in Alzheimer's disease - current concepts. Mol. Neurodegener. 8, 20.
17 Gabelle A, Touchon J & Lehmann S (2013) Les biomarqueurs du LCR et du plasma : utilisation diagnostique et pronostique dans la maladie d'Alzheimer et les syndromes apparentés. Prat. Neurol. - FMC 4, 65-72.
18 Laxman Kole P, Venkatesh G, Kotecha J & Sheshala R (2011) Recent advances in sample preparation techniques for effective bioanalytical methods. Biomed. Chromatogr. 25, 199-217.
19 Sabbagh MN, Fleisher A, Chen K, Rogers J, Berk C, Reiman E, Pontecorvo M, Mintun M, Skovronsky D, Jacobson SA, Sue LI, Liebsack C, Charney AS, Cole L, Belden C & Beach TG (2011) Positron emission tomography and neuropathologic estimates of fibrillar amyloid-β in a patient with Down syndrome and Alzheimer disease. Arch. Neurol. 68, 1461-6.
20 Babu SVS, Shareef MM, Shetty APK & Shetty KT (2002) HPLC method for amino acids profile in biological fluids and inborn metabolic disorders of aminoacidopathies. Indian J. Clin. Biochem. 17, 7-26.
21 Lima E.S. & Abdalla D.S.P. High-performance liquid chromatography of fatty acids in biological samples. Anal. Chim. Acta 465, 11.
22 Chen S.-H. & Chuang Y.-J. Analysis of fatty acids by column liquid chromatography. Anal. Chim. Acta 465, 11.
23 Bondia-Pons I, Castellote AI & López-Sabater MC (2004) Comparison of conventional and fast gas chromatography in human plasma fatty acid determination. J. Chromatogr. B. Analyt. Technol. Biomed. Life Sci. 809, 339-44.
24 Stoltenburg R, Reinemann C & Strehlitz B (2007) SELEX--a (r)evolutionary method to generate high-affinity nucleic acid ligands. Biomol. Eng. 24, 381-403.
25 Radom F, Jurek PM, Mazurek MP, Otlewski J & Jelen F (2013) Aptamers: Molecules of great potential. Biotechnol. Adv. 31, 1260-1274.
26 Hsiao K, Chapman P, Nilsen S, Eckman C, Harigaya Y, Younkin S, Yang F & Cole G (1996) Correlative memory deficits, Abeta elevation, and amyloid plaques in transgenic mice. Science 274, 99-102.
27 Guedj M, Robin S, Celisse A & Nuel G (2009) Kerfdr: a semi-parametric kernel-based approach to local false discovery rate estimation. BMC Bioinformatics 10, 84.
28 Kuhn A, Luthi-Carter R & Delorenzi M (2008) Cross-species and cross-platform gene expression studies with the Bioconductor-compliant R package "annotationTools". BMC Bioinformatics 9, 26.
29 McLachlan GJ (2004) Discriminant Analysis and Statistical Pattern Recognition (J. Wiley, ed.) Wiley Interscience.

## Claims

1. An *in vitro* method for diagnosing a neurological disease selected from Alzheimer's disease (AD), senile dementia of AD type (SDAT), prodromal AD, mild cognitive impairment (MCI), age associated memory impairment (AAMI), vascular dementia or frontotemporal dementia (FTD) in a subject, the method comprising determining the presence, quantity, frequency or form of sebacic acid, in a sample of blood, serum and/or plasma from said subject, wherein an alteration in the presence, quantity, frequency or form of sebacic acid, as compared to a control, is indicative of the presence, risk, subtype, progression or severity of said disease.

2. The *in vitro* method of claim 1, comprising determining simultaneously or sequentially the presence of an alteration in the quantity, frequency or form of a combination of at least four biomarkers comprising sebacic acid, dodecanedioic acid, tryptophan and at least one metabolite selected from PFAM (20:1), PFAM (22:1), PFAM (22:2), azelaic acid, hippuric acid, tyrosine, 4-methyl-2-oxovaleric acid, caffeine, caproicacid, isovaleric acid, L-citrulline, phenylacetylglutamine, C7H8N4O2(theophylline and/or paraxanthine), valeric acid, aminoisobutyric acid, aspartate, Asp-Phe, glycocholic acid, guanosine, inosine, L-threonic acid, undecanedioic acid, 1-monopalmitin, 9,12-dioxododecanoic acid, nonenedioic acid, octadecadienoyl-glycero-3-phosphate, Ser-Phe and sulfobenzylalcohol.

3. The *in vitro* method of claim 2, wherein an increase of sebacic acid and a decrease of at least one biomarker selected from caffeine, glycocholic acid, guanosine, hippuric acid, inosine, L-citrulline, L-threonic acid, PFAM (22:1), tryptophan, tyrosine, 4-methyl-2-oxovaleric acid, PFAM (20:1), PFAM (22:2), Ser-Phe, C7H8N4O2(theophylline and/or paraxanthine), or valeric acid is indicative of the presence, risk, subtype, progression or severity of said disease.

4. The *in vitro* method of claims 1 or 2, comprising determining simultaneously or sequentially the presence of an alteration in the quantity, frequency or form of a set of biomarkers selected from:
- Asp-Phe and C₇H₈N₄O₂ (theophylline and/or paraxanthine) and L-threonic acid and sebacic acid,
- L-citrulline and hippuric acid and sebacic acid and dodecanedioic acid and tryptophan,
- Azelaic acid and sebacic acid,
- Azelaic acid and sebacic acid and dodecanedioic acid,
- Azelaic acid and undecanedioic acid and sebacic acid,
- Azelaic acid and undecanedioic acid and sebacic acid and dodecanedioic acid,
- Nonenedioic acid and sebacic acid,
- Nonenedioic acid and azelaic acid and sebacic acid,
- Nonenedioic acid and sebacic acid and dodecanedioic acid,
- Nonenedioic acid and azelaic acid and sebacic acid and dodecanedioic acid,
- Nonenedioic acid and azelaic acid and undecanedioic acid and sebacic acid,
- Nonenedioic acid and azelaic acid and undecanedioic acid and sebacic acid and
- dodecanedioic acid,
- Undecanedioic acid and sebacic acid,
- Nonenedioic acid and undecanedioic acid and sebacic acid,
- Nonenedioic acid and Sebacic acid and Dodecanedioic acid,
- Nonenedioic acid and undecanedioic acid and sebacic acid and dodecanedioic acid,
- Undecanedioic acid and sebacic acid and dodecanedioic acid,
- Sebacic acid and tyrosine,
- Dodecanedioic acid and sebacic acid and tyrosine,
- Dodecanedioic acid and sebacic acid and tyrosine and undecanedioic acid
- Dodecanedioic acid and hippuric acid and sebacic acid and tyrosine,
- Sebacic acid and tryptophan and tyrosine,
- Sebacic acid and tryptophan,
- Sebacic acid and tryptophan and undecanedioic acid,
- Sebacic acid and Tyrosine and Undecanedioic acid,
- Sebacic acid and tryptophan and tyrosine and undecanedioic acid
- Hippuric acid and sebacic acid and tyrosine and undecanedioic acid,
- Hippuric acid and sebacic acid and tryptophan and tyrosine,
- Dodecanedioic acid and sebacic acid and tryptophan,
- Dodecanedioic acid and sebacic acid and tryptophan and tyrosine
- Hippuric acid and sebacic acid,
- Sebacic acid and tryptophan and tyrosine and undecanedioic acid,
- Hippuric acid and sebacic acid and tryptophan and undecanedioic acid,
- Hippuric acid and sebacic acid and tryptophan,
- Hippuric acid and sebacic acid and tyrosine,
- Dodecanedioic acid and sebacic acid and tryptophan and undecanedioic acid,
- Hippuric acid and sebacic acid and tryptophan and tyrosine and undecanedioic acid,
- Dodecanedioic acid and sebacic acid and tryptophan and tyrosine and undecanedioic acid,
- Dodecanedioic acid and hippuric acid and sebacic acid and tryptophan and tyrosine,
- Dodecanedioic acid and hippuric acid and sebacic acid,
- Hippuric acid and sebacic acid and undecanedioic acid,
- Dodecanedioic acid and hippuric acid and sebacic acid and tryptophan,
- Dodecanedioic acid and hippuric acid and sebacic acid and undecanedioic acid,
- Dodecanedioic acid and hippuric acid and sebacic acid and tryptophan and tyrosine and undecanedioic acid,
- Dodecanedioic acid and sebacic acid,
- Dodecanedioic acid and hippuric acid and sebacic acid and tryptophan and undecanedioic acid,
- C7H8N4O2 (theophylline and/or paraxanthine) and Asp-Phe and L-threonic acid and sebacic acid.

5. The *in vitro* method of any one of the preceding claims, further comprising the simultaneous or sequential determination of an alteration in the quantity, frequency or form of at least one additional biomarker or diagnostic test.

6. The method of claim 5, wherein the at least one additional diagnostic test or biomarker is selected from nucleic acids, proteins, metabolites, neurophysiological, genetic, brain imaging, clinical and cognitive tests or markers.

7. An *in vitro* method for assessing the responsiveness of a subject to a treatment for a neurological disease selected from Alzheimer's disease (AD), senile dementia of AD type, prodromal AD, mild cognitive impairment, age associated memory impairment, vascular dementia or frontotemporal dementia, the method comprising determining in blood, serum and/or plasma sample from said subject, the presence, quantity, frequency or form, of one or more biomarker(s) as defined in any one of claims 1 to 6, during the course of said treatment, wherein an alteration in said presence, quantity, frequency or form is indicative of a subject responsive to a treatment for said disease.

8. An *in vitro* method for monitoring the effect of a treatment in a subject having a neurological disease selected from Alzheimer's disease (AD), senile dementia of AD type, prodromal AD, mild cognitive impairment, age associated memory impairment, vascular dementia or frontotemporal dementia, the method comprising determining an alteration of the quantity, frequency or form, as compared to a control, in blood, serum and/or plasma sample from the subject, of one or more biomarker(s) as defined in any one of claims 1 to 6, after the administration of said treatment and/or at different point of times during the course of the treatment, wherein a correction of such alteration during treatment is indicative of an effective treatment.

9. An *in vitro* method for diagnosing a neurological disease selected from Alzheimer's disease (AD), senile dementia of AD type, prodromal AD, mild cognitive impairment, age associated memory impairment, vascular dementia or frontotemporal dementia, said method comprising the following steps:
- collecting a blood, serum or plasma sample from a subject suffering from, or suspected to suffer from, or at risk of suffering from said disease,
- treating the sample for further analysis by LC/MS and/or GC/MS, and
- measuring by LC/MS and/or GC/MS the amount of sebacic acid in said treated sample, wherein an increase in sebacic acid as compared to a control value is indicative of the presence, risk, subtype, progression or severity of said disease.

10. The *in vitro* method according to claim 9, further comprising determining from a sample the amount of at least one additional biomarker selected from caffeine, glycocholic acid, guanosine, hippuric acid, inosine, L-citrulline, L-threonic acid, PFAM (22:1), tryptophan, tyrosine, 4-methyl-2-oxovaleric acid, PFAM (20:1), PFAM (22:2), Ser-Phe, C7H8N4O2 (theophylline and/or paraxanthine), or valeric acid, by LC/MS and/or GC/MS, wherein a decrease in said amount, as compared to a control value, is indicative of the presence, risk, subtype, progression or severity of said disease.

11. *The in vitro* method of any one of the preceding claims, wherein sebacic acid concentration is increased in diseased subjects from 10 to 90%, preferably from 30% to 70%, and more preferably of 50%, as compared to a concentration level in a control sample.

12. The *in vitro* method of any one of claims 2 to 8 or 10 to 11, wherein dodecanedioic acid concentration is increased in diseased subjects from 10 to 90%, preferably from 40% to 80%, and more preferably of 60%, as compared to a concentration level in a control sample.

13. The *in vitro* method of any one of claims 2 to 8 or 10 to 12, wherein tryptophan concentration is decreased in diseased subjects from 10 to 90%, preferably from 10% to 50%, and more preferably of 20%, as compared to a concentration level in a control sample.

14. The *in vitro* method of any one of the preceding claim, wherein the control is the quantity and/or the frequency and/or the form of the biomarker as determined in a similar sample from a healthy subject, or a reference value, and/or level(s) of the biomarker in a sample from the same subject before disease development and/or at an earlier stage of treatment/disease in the subject.

## Patentansprüche

1. Ein *in vitro* Verfahren zur Diagnose einer neurologischen Erkrankung ausgewählt aus Alzheimer Krankheit (AD), seniler Demenz vom Typ AD (SDAT), prodromaler AD, milder kognitiver Beeinträchtigung (MCI), altersbedingter Gedächtnisstörung (AAMI), vaskulärer Demenz oder frontotemporaler Demenz (FTD) bei einem Subjekt, wobei das Verfahren das Bestimmen des Vorhandensein, der Menge, der Häufigkeit oder der Form von Sebacinsäure in einer Blut-, Serum- und/oder Plasmaprobe von dem Subjekt umfasst, wobei eine Änderung in dem Vorhandensein, der Menge, der Häufigkeit oder der Form der Sebacinsäure im Vergleich zu einer Kontrolle ein Anzeichen für das Vorliegen, das Risiko, den Subtyp, das Fortschreiten oder der Schwere der Erkrankung ist.

2. Das *in vitro* Verfahren nach Anspruch 1, umfassend das gleichzeitige oder sequentielle Bestimmen des Vorliegens einer Änderung in der Menge, der Häufigkeit oder der Form einer Kombination von mindestens vier Biomarkern umfassend Sebacinsäure, Dodecandisäure, Tryptophan und mindestens einen Metaboliten ausgewählt aus PFAM (20:1), PFAM (22:1), PFAM (22:2), Azelainsäure, Hippursäure, Tyrosin, 4-Methyl-2-oxovaleriansäure, Koffein, Caprosäuren, Isovaleriansäure, L-Citrullin, Phenylacetylglutamin, C7H8N4O2 (Theophyllin und/oder Paraxanthin), Valeriansäure, Aminoisobuttersäure, Aspartat, Asp-Phe, Glykocholsäure, Guanosin, Inosin, L-Threonsäure, Undecansäure, 1-Monopalmitin, 9,12-Dioxododekansäure, Nonendisäure, Octadecadienoyl-glycero-3-phosphat, Ser-Phe und Sulfobenzylalkohol.

3. Das *in vitro* Verfahren nach Anspruch 2, wobei eine Erhöhung der Sebacinsäure und eine Verminderung mindestens eines Biomarkers ausgewählt aus Koffein, Glykocholsäure, Guanosin, Hippursäure, Inosin, L-Citrullin, L-Threonsäure, PFAM (22:1), Tryptophan, Tyrosin, 4-Methyl-2-oxovaleriansäure, PFAM (20:1), PFAM (22:2), Ser-Phe, C7H8N4O2 (Theophyllin und/oder Paraxanthin) oder Valeriansäure ein Anzeichen für das Vorliegen, das Risiko, den Subtyp, das Fortschreiten oder die Schwere der Krankheit ist.

4. Das *in vitro* Verfahren nach den Ansprüchen 1 oder 2, umfassend das gleichzeitige oder sequentielle Bestimmen des Vorliegens einer Änderung in der Menge, der Häufigkeit oder der Form eines Satzes von Biomarker ausgewählt aus:
- Asp-Phe und C₇H₈N₄O₂ (Theophyllin und/oder Paraxanthin) und L-Threonsäure und Sebacinsäure,
- L-Citrullin und Hippursäure und Sebacinsäure und Dodecandisäure und Tryptophan,
- Azelainsäure und Sebacinsäure,
- Azelainsäure und Sebacinsäure und Dodecandisäure,
- Azelainsäure und Undecandisäure und Sebacinsäure,
- Azelainsäure und Undecandisäure und Sebacinsäure und Dodecandisäure,
- Nonendisäure und Sebacinsäure,
- Nonendisäure und Azelainsäure und Sebacinsäure,
- Nonendisäure und Sebacinsäure und Dodecandisäure,
- Nonendisäure und Azelainsäure und Sebacinsäure und Dodecandisäure,
- Nonendisäure und Azelainsäure und Undecandisäure und Sebacinsäure,
- Nonendisäure und Azelainsäure und Undecandisäure und Sebacinsäure und
- Dodecandisäure,
- Undecandisäure und Sebacinsäure,
- Nonendisäure und Undecandisäure und Sebacinsäure,
- Nonendisäure und Sebacinsäure und Dodecandisäure,
- Nonendisäure und Undecandisäure und Sebacinsäure und Dodecandisäure,
- Undecandisäure und Sebacinsäure und Dodecandisäure,
- Sebacinsäure und Tyrosin,
- Dodecandisäure und Sebacinsäure und Tyrosin,
- Dodecandisäure und Sebacinsäure und Tyrosin und Undekandisäure
- Dodecandisäure und Hippursäure und Sebacinsäure und Tyrosin,
- Sebacinsäure und Tryptophan und Tyrosin,
- Sebacinsäure und Tryptophan,
- Sebacinsäure und Tryptophan und Undecandisäure,
- Sebacinsäure und Tyrosin und Undecandisäure,
- Sebacinsäure und Tryptophan und Tyrosin und Undecandisäure
- Hippursäure und Sebacinsäure und Tyrosin und Undecandisäure,
- Hippursäure und Sebacinsäure und Tryptophan und Tyrosin,
- Dodecandisäure und Sebacinsäure und Tryptophan,
- Dodecandisäure und Sebacinsäure und Tryptophan und Tyrosin
- Hippursäure und Sebacinsäure,
- Sebacinsäure und Tryptophan und Tyrosin und Undecandisäure,
- Hippursäure und Sebacinsäure und Tryptophan und Undecandisäure,
- Hippursäure und Sebacinsäure und Tryptophan,
- Hippursäure und Sebacinsäure und Tyrosin,
- Dodecandisäure und Sebacinsäure und Tryptophan und Undecandisäure,
- Hippursäure und Sebacinsäure und Tryptophan und Tyrosin und Undecandisäure,
- Dodecandisäure und Sebacinsäure und Tryptophan und Tyrosin und Undekandisäure,
- Dodecandisäure und Hippursäure und Sebacinsäure und Tryptophan und Tyrosin,
- Dodecandisäure und Hippursäure und Sebacinsäure,
- Hippursäure und Sebacinsäure und Undecandisäure,
- Dodecandisäure und Hippursäure und Sebacinsäure und Tryptophan,
- Dodecandisäure und Hippursäure und Sebacinsäure und Undecandisäure,
- Dodecandisäure und Hippursäure und Sebacinsäure und Tryptophan und Tyrosin und Undecandisäure,
- Dodecandisäure und Sebacinsäure,
- Dodecandisäure und Hippursäure und Sebacinsäure und Tryptophan und Undekandisäure,
- C7H8N4O2 (Theophyllin und/oder Paraxanthin) und Asp-Phe und L-Threonsäure und Sebacinsäure.

5. Das *in vitro* Verfahren nach einem der vorhergehenden Ansprüche, ferner umfassend die gleichzeitige oder sequentielle Bestimmung einer Änderung in der Menge, der Häufigkeit oder der Form von mindestens einem zusätzlichen Biomarkers oder diagnostischen Test.

6. Das Verfahren nach Anspruch 5, wobei der mindestens eine zusätzliche diagnostische Test oder Biomarker ausgewählt ist aus Nukleinsäuren, Proteinen, Metaboliten, neurophysiologischen, genetischen, gehirnbildgebenden, klinischen und kognitiven Test oder Marker.

7. Ein *in vitro* Verfahren zur Beurteilung der Ansprechbarkeit eines Subjekts auf eine Behandlung einer neurologischen Erkrankung ausgewählt aus Alzheimer Krankheit (AD), seniler Demenz vom Typ AD, prodromaler AD, milder kognitiver Beeinträchtigung, altersbedingter Gedächtnisstörung, vaskulärer Demenz oder frontotemporaler Demenz, wobei das Verfahren das Bestimmen des Vorhandensein, der Menge, der Häufigkeit oder der Form eines oder mehrerer Biomarker(s) wie in einem der Ansprüche 1 bis 6 definiert in einer Blut-, Serum- und/oder Plasmaprobe des Subjekts während der Behandlung umfasst, wobei eine Änderung in dem Vorhandensein, der Menge, der Häufigkeit oder der Form ein Anzeichen für ein Ansprechen des Subjekts auf die Behandlung der Erkrankung ist.

8. Ein *in vitro* Verfahren zur Überwachung der Wirkung einer Behandlung bei einem Subjekt mit einer neurologische Erkrankung ausgewählt aus Alzheimer Krankheit (AD), seniler Demenz vom Typ AD, prodromaler AD, milder kognitiver Beeinträchtigung, altersbedingter Gedächtnisstörung, vaskulärer Demenz oder frontotemporaler Demenz, wobei das Verfahren das Bestimmen einer Änderung der Menge, der Häufigkeit oder der Form von einem oder mehreren Biomarker(n) wie in einem der Ansprüche 1 bis 6 definiert in einer Blut-, Serum- und/oder Plasmaprobe von dem Subjekt nach der Verabreichung der Behandlung und/oder zu verschiedenen Zeitpunkten während der Behandlung im Vergleich zu einer Kontrolle umfasst, wobei eine Korrektur einer solchen Änderung während der Behandlung ein Anzeichen für eine effektive Behandlung ist.

9. Ein *in vitro* Verfahren zur Diagnose einer neurologischen Erkrankung ausgewählt aus Alzheimer Krankheit (AD), seniler Demenz vom Typ AD, prodromaler AD, milder kognitiver Beeinträchtigung, altersbedingter Gedächtnisstörung, vaskulärer Demenz oder frontotemporaler Demenz, das Verfahren umfasst die folgenden Schritte:
- Entnahme einer Blut-, Serum- oder Plasmaprobe von einem Subjekt, das an dieser Erkrankung leidet, unter Verdacht steht, darunter zu leiden oder ein Risiko aufweist, darunter zu leiden,
- Behandlung der Probe zur weiteren Analyse mittels LC/MS und/oder GC/MS und
- Messen der Menge an Sebacinsäure in der behandelten Probe mittels LC/MS und/oder GC/MS,
wobei eine Erhöhung der Sebacinsäure im Vergleich zu einem Kontrollwert ein Anzeichen für das Vorliegen, das Risiko, den Subtyp, den Fortschritt oder die Schwere der Erkrankung ist.

10. Das *in vitro* Verfahren nach Anspruch 9, ferner umfassend das Bestimmen der Menge von mindestens einem zusätzlichen Biomarker ausgewählt aus Koffein, Glykocholsäure, Guanosin, Hippursäure, Inosin, L-Citrullin, L-Threonsäure, PFAM (22:1), Tryptophan, Tyrosin, 4-Methyl-2-oxovaleriansäure, PFAM (20:1), PFAM (22:2), Ser-Phe, C7H8N4O2 (Theophyllin und/oder Paraxanthin) oder Valeriansäure, durch LC/MS und/oder GC/MS in einer Probe, wobei eine Verringerung der Menge, verglichen mit einem Kontrollwert, ein Anzeichen für das Vorliegen, das Risiko, den Subtyp, den Fortschritts oder die Schwere der Erkrankung ist.

11. Das *in vitro* Verfahren nach einem der vorhergehenden Ansprüche, wobei die Sebacinsäurekonzentration bei erkrankten Subjekten von 10 bis 90%, vorzugsweise von 30% bis 70%, und noch bevorzugter um 50% im Vergleich zu einem Konzentrationsniveau einer Kontrollprobe erhöht ist.

12. Das *in vitro* Verfahren nach einem der Ansprüche 2 bis 8 oder 10 bis 11, wobei die Dodecandisäurekonzentration bei erkrankten Subjekten von 10 bis 90%, vorzugsweise von 40% bis 80%, und noch bevorzugter um 60% im Vergleich zu einem Konzentrationsniveau einer Kontrollprobe erhöht ist.

13. Das *in vitro* Verfahren nach einem der Ansprüche 2 bis 8 oder 10 bis 11, wobei die Tryptophankonzentration bei erkrankten Subjekten von 10 bis 90%, vorzugsweise von 10% bis 50%, und noch bevorzugter um 20% im Vergleich zu einem Konzentrationsniveau einer Kontrollprobe vermindert ist.

14. Das *in vitro* Verfahren nach einem der vorhergehenden Ansprüche, wobei die Kontrolle die Menge und/oder die Häufigkeit und/oder die Form des Biomarkers wie in einer ähnlichen Probe von einem gesunden Subjekt bestimmt ist oder ein Referenzwert und/oder das/die Niveau(s) des Biomarkers in einer Probe von dem gleichen Subjekt vor der Entwicklung der Erkrankung und/oder eines früheren Stadiums der Behandlung/Erkrankung in dem Subjekt.

## Revendications

1. Méthode *in vitro* pour diagnostiquer une maladie neurologique choisie parmi la maladie d'Alzheimer (MA), la démence sénile de type Alzheimer (DSTA), la maladie d'Alzheimer prodromique, le déficit cognitif léger (DCL), les troubles de la mémoire liés à l'âge (TMLA), la démence vasculaire ou la démence frontotemporale (DFT) chez un sujet, la méthode comprenant la détermination de la présence, de la quantité, de la fréquence ou de la forme de l'acide sébacique dans un échantillon de sang, de sérum et/ou de plasma dudit sujet, dans laquelle un changement dans la présence, la quantité, la fréquence ou la forme de l'acide sébacique, par rapport à un contrôle, est indicatif de la présence, du risque, du sous-type, de la progression ou de la sévérité de ladite maladie.

2. Méthode *in vitro* selon la revendication 1, comprenant la détermination simultanément ou séquentiellement de la présence d'un changement dans la quantité, la fréquence ou la forme d'une combinaison d'au moins quatre biomarqueurs comprenant l'acide sébacique, l'acide dodécanédioïque, le tryptophane et au moins un métabolite choisi parmi PFAM (20:1), PFAM (22:1), PFAM (22:2), l'acide azélaïque, l'acide hippurique, la tyrosine, l'acide 4-méthyle-2-oxovalérique, la caféine, l'acide caproique, l'acide isovalérique, la L-citrulline, la phénylacétylglutamine, C7H8N4O2 (la théophylline et/ou la paraxanthine), l'acide valérique, l'acide aminoisobutyrique, l'aspartate, Asp-Phe, acide glycocholique, la guanosine, l'inosine, l'acide de L-thréonique, l'acide undecanédioïque, la 1-monopalmitine, l'acide 9,12-dioxo-dodécanoïque, l'acide nonenedioïque, l'octadécadienoyl-glycéro-3-phosphate, Ser-Phe et le sulfobenzylalcool.

3. Méthode *in vitro* selon la revendication 2, dans laquelle une augmentation de l'acide sébacique et une diminution d'au moins un biomarqueur choisi parmi la caféine, l'acide glycocholique, la guanosine, l'acide hippurique, l'inosine, la L-citrulline, l'acide L-thréonique, PFAM (22:1), le tryptophane, la tyrosine, l'acide 4-méthyle-2-oxovalérique, PFAM (20:1), PFAM (22:2), Ser-Phe, C7H8N4O2 (la théophylline et/ou la paraxanthine), ou l'acide valérique est indicative de la présence, du risque, du sous-type, de la progression ou de la sévérité de ladite maladie.

4. Méthode *in vitro* selon la revendication 1 ou 2, comprenant la détermination simultanément ou séquentiellement de la présence d'un changement dans la quantité, la fréquence ou la forme d'un ensemble de biomarqueurs choisi parmi:
- Asp-Phe et C₇H₈N₄O₂ (théophylline et/ou paraxanthine) et acide L-thréonique et acide sébacique,
- L-citrulline et acide hippurique et acide sébacique et acide dodécanédioïque et tryptophane,
- Acide azélaïque et acide sébacique,
- Acide azélaïque et acide sébacique et acide dodécanédioïque,
- Acide azélaïque et acide undecanédioïque et acide sébacique,
- Acide azélaïque et acide undecanédioïque et acide sébacique et acide dodécanédioïque,
- Acide nonenedioïque et acide sébacique,
- Acide nonenedioïque et acide azélaïque et acide sébacique,
- Acide nonenedioïque et acide sébacique et acide dodécanédioïque,
- Acide nonenedioïque et acide azélaïque et acide sébacique et acide dodécanédioïque,
- Acide nonenedioïque et acide azélaïque et acide undecanédioïque et acide sébacique,
- Acide nonenedioïque et acide azélaïque et acide undecanédioïque et acide sébacique et acide dodécanédioïque,
- Acide undecanédioïque et acide sébacique,
- Acide nonenedioïque et acide undecanédioïque et acide sébacique,
- Acide nonenedioïque et acide sébacique et acide dodécanédioïque,
- Acide nonenedioïque et acide undecanédioïque et acide sébacique et acide dodécanédioïque,
- Acide undecanédioïque et acide sébacique et acide dodécanédioïque,
- Acide sébacique et tyrosine,
- Acide dodécanédioïque et acide sébacique et tyrosine,
- Acide dodécanédioïque et acide sébacique et tyrosine et acide undecanédioïque,
- Acide dodécanédioïque et acide hippurique et acide sébacique et tyrosine,
- Acide sébacique et tryptophane et tyrosine,
- Acide sébacique et tryptophane,
- Acide sébacique et tryptophane et acide undecanédioïque,
- Acide sébacique et tyrosine et acide undecanédioïque,
- Acide sébacique et tryptophane et tyrosine et acide undecanédioïque,
- Acide hippurique et acide sébacique et tyrosine et acide undecanédioïque,
- Acide hippurique et acide sébacique et tryptophane et tyrosine,
- Acide dodécanédioïque et acide sébacique et tryptophane,
- Acide dodécanédioïque et acide sébacique et tryptophane et tyrosine
- Acide hippurique et acide sébacique,
- Acide sébacique et tryptophane et tyrosine et acide undecanédioïque,
- Acide hippurique et acide sébacique et tryptophane et acide undecanédioïque,
- Acide hippurique et acide sébacique et tryptophane,
- Acide hippurique et acide sébacique et tyrosine,
- Acide dodécanédioïque et acide sébacique et tryptophane et acide undecanédioïque,
- Acide hippurique et acide sébacique et tryptophane et tyrosine et acide undecanédioïque,
- Acide dodécanédioïque et acide sébacique et tryptophane et tyrosine et acide undecanédioïque,
- Acide dodécanédioïque et acide hippurique et acide sébacique et tryptophane et tyrosine,
- Acide dodécanédioïque et acide hippurique et acide sébacique,
- Acide hippurique et acide sébacique et acide undecanédioïque,
- Acide dodécanédioïque et acide hippurique et acide sébacique et tryptophane,
- Acide dodécanédioïque et acide hippurique et acide sébacique et acide undecanédioïque,
- Acide dodécanédioïque et acide hippurique et acide sébacique et tryptophane et tyrosine et acide undecanédioïque,
- Acide dodécanédioïque et acide sébacique,
- Acide dodécanédioïque et acide hippurique et acide sébacique et tryptophane et acide undecanédioïque,
- C7H8N4O2 (théophylline et/ou paraxanthine) et Asp-Phe et acide L-thréonique et acide sébacique.

5. Méthode *in vitro* selon l'une quelconque des revendications précédentes, comprenant en outre la détermination simultanée ou séquentielle d'un changement dans la quantité, la fréquence ou la forme d'au moins un biomarqueur ou un test de diagnostic supplémentaire.

6. Méthode selon la revendication 5, dans laquelle le au moins un test de diagnostic ou biomarqueur supplémentaire est choisi parmi des acides nucléiques, des protéines, des métabolites, des tests ou marqueurs neurophysiologiques, génétiques, d'imagerie cérébrale, cliniques et cognitifs.

7. Méthode *in vitro* pour évaluer la réponse d'un sujet à un traitement pour une maladie neurologique choisie parmi la maladie d'Alzheimer (MA), la démence sénile de type Alzheimer, la maladie d'Alzheimer prodromique, le déficit cognitif léger, les troubles de la mémoire liés à l'âge, la démence vasculaire ou la démence frontotemporale, la méthode comprenant la détermination dans un échantillon de sang, de sérum et/ou de plasma dudit sujet, de la présence, de la quantité, de la fréquence ou de la forme d'un ou plusieurs biomarqueur(s) comme défini dans l'une quelconque des revendications 1 à 6, pendant ledit traitement, où un changement dans ladite présence, quantité, fréquence ou forme est indicatif d'un sujet répondant au traitement pour ladite maladie.

8. Méthode *in vitro* pour surveiller l'effet d'un traitement chez un sujet ayant une maladie neurologique choisie parmi la maladie d'Alzheimer (MA), la démence sénile de type Alzheimer, la maladie d'Alzheimer prodromique, le déficit cognitif léger, les troubles de la mémoire liés à l'âge, la démence vasculaire ou la démence frontotemporale, la méthode comprenant la détermination d'un changement dans la quantité, la fréquence ou la forme, par rapport à un contrôle, dans échantillon de sang, du sérum et/ou de plasma à partir du sujet, d'un ou plusieurs biomarqueur(s) comme défini dans l'une quelconque des revendications 1 à 6, après l'administration dudit traitement et/ou à différents moments du traitement, où une correction d'un tel changement pendant le traitement est indicative d'un traitement efficace.

9. Méthode *in vitro* pour diagnostiquer une maladie neurologique choisie parmi la maladie d'Alzheimer (MA), la démence sénile de type Alzheimer, la maladie d'Alzheimer prodromique, le déficit cognitif léger, les troubles de la mémoire liés à l'âge, la démence vasculaire ou la démence frontotemporale, ladite méthode comprenant les étapes suivantes :
- la collecte d'un échantillon de sang, de sérum ou de plasma à partir d'un sujet souffrant de, ou suspecté de souffrir de, ou à risque de souffrir de ladite maladie,
- le traitement de l'échantillon pour une analyse ultérieure par LC/MS et/ou GC/MS, et
- la mesure par LC/MS et/ou GC/MS de la quantité de l'acide sébacique dans ledit échantillon traité, où une augmentation de l'acide sébacique par rapport à une valeur de contrôle est indicative de la présence, du risque, du sous-type, de la progression ou de la sévérité de ladite maladie.

10. Méthode *in vitro* selon la revendication 9, comprenant en outre la détermination à partir d'un échantillon de la quantité d'au moins un biomarqueur supplémentaire choisi parmi la caféine, l'acide glycocholique, la guanosine, l'acide hippurique, l'inosine, la L-citrulline, l'acide L-thréonique, PFAM (22:1), le tryptophane, la tyrosine, l'acide 4-méthyle-2-oxovalérique, PFAM (20:1), PFAM (22:2), Ser-Phe, C7H8N4O2 (théophylline et/ou paraxanthine), ou l'acide valérique, par LC/MS et/or GC/MS, où une diminution dans ladite quantité, par rapport à une valeur de contrôle, est indicative de la présence, du risque, du sous-type, de la progression ou de la sévérité de ladite maladie.

11. Méthode *in vitro* selon l'une quelconque des revendications précédentes, où la concentration d'acide sébacique est augmentée dans les sujets malades de 10 à 90%, de préférence de 30% à 70%, et plus préférentiellement de 50%, par rapport à un niveau de concentration dans un échantillon de contrôle.

12. Méthode *in vitro* selon l'une quelconque des revendications 2 à 8 ou 10 à 11, où la concentration d'acide dodécanédioïque est augmentée chez les sujets malades de 10 à 90%, de préférence de 40% à 80%, et plus préférentiellement de 60%, par rapport à un niveau de concentration dans un échantillon de contrôle.

13. Méthode *in vitro* selon l'une quelconque des revendications 2 à 8 ou 10 à 12, où la concentration de tryptophane est diminuée chez les sujets malades de 10 à 90%, de préférence de 10% à 50%, et plus préférentiellement de 20%, par rapport à un niveau de concentration dans un échantillon de contrôle.

14. Méthode *in vitro* selon l'une quelconque des revendications précédentes, dans laquelle le contrôle est la quantité et/ou la fréquence et/ou la forme du biomarqueur comme déterminé dans un échantillon semblable à partir d'un sujet sain, ou une valeur de référence, et/ou un/des niveau(x) du biomarqueur dans un échantillon à partir du même sujet avant le développement de la maladie et/ou à un stade antérieur de traitement/maladie chez le sujet.
